# EUROPEAN PATENT APPLICATION

(11) **EP 4 455 155 A1**
(43) Date of publication of application: **30.10.2024**
(21) Application number: 23742979.0
(22) Date of filing: 19.01.2023
(51) Int. Cl.: C07H 15/26, C07H 21/00, A61K 31/713, A61P 1/16, C12N 15/11

(54) **TARGETING LIGAND AND USE THEREOF**

(30) Priority: 20.01.2022 CN 202210068123
(71) Applicant: Chengdu Xinzhenghe Pharmaceutical Technology Co. Ltd, Sichuan 610041 (CN)
(72) Inventor: LI, Chong, Shanghai 200120 (CN); YIN, Ke, Shanghai 200120 (CN); LI, Qian, Shanghai 200120 (CN); MA, Haiping, Shanghai 200120 (CN)
(74) Representative: Fuchs Patentanwälte Partnerschaft mbB
(86) International application number: PCT/CN2023/073193
(87) International publication number: WO 2023/138664

(57) **Abstract**

The present invention is suitable for the technical field of nucleic acid drug delivery; specifically provided are a nucleic acid delivery compound, a liver-targeting nucleic acid ligand and the use thereof. Provided in the present invention are a compound as represented by formula (Z-1), and compounds as represented by formula (I) and (II). An oligonucleotide conjugate provided in the embodiments of the present invention can improve the binding efficiency of a targeting moiety to a cell or a cell receptor, and increase the effect of RNA interference.

## Description

### Cross Reference

The present application is based upon and claims the benefit of priority of Chinese patent application number 202210068123.3, filed on January 20, 2022, titled **siRNA Conjugate and Its Uses,** the entire content of which is incorporated herein by reference.

### Technical Field

The present invention relates to the field of nucleic acid drug delivery technology, in particular to a kind of nucleic acid delivery compounds, liver-targeted nucleic acid ligands, and their uses.

### Background

RNA interference (RNAi), which is widely present in nature, has developed into a mature technology in the fields of molecular biology and medicine.

Targeting ligand, a crucial part of the RNAi process, assists in guiding the delivery of conjugated therapeutic reagents to the target location, and the targeting portion of targeting ligand consists of one or more targeting groups or portions. In some cases, the targeting portion can bind to a cell or a cell receptor and initiate endocytosis to promote the entry of therapeutic agents into cells.

The liver is an important organ for human metabolism, and hepatocytes, the major cells in the liver that perform liver functions, have abundant asialoglycoprotein receptors (ASGPRs) on the surface. It is known that after the combination with ASGPR, the targeting portion can be specifically used to deliver oligonucleotide compounds (for example, siRNA and the like) to the liver. The targeting portion of ASGPR includes galactose or galactose derivatives. During use, N-acetyl galactosamine (GalNAc) clusters conjugated with oligonucleotides compounds are used to guide the oligonucleotides to the liver, where GalNAc can bind to ASGPR on the hepatocytes surface, thereby promoting the entry of the compound into the cell interior. Therefore improving this targeting ligand can enhance the working efficiency of RNAi.

Since the nucleoside molecule generally has high negative charges, on the other hand, the complex system of cell membrane limits the direct diffusion of nucleic acid or other compounds that can hardly penetrate the cell membrane into living cells. As a result, the main obstacle to the delivery of nucleic acid molecules is how to deliver them to the cytoplasm or nucleus. At present, published solutions include the use of nanoliposomes, viral carriers, conjugated delivery molecules, etc. Conjugated delivery molecules, such as GalNAc molecules, peptide molecules, antibody molecules, etc., serve as guides to lead siRNA to targeting cells. It is therefore required to develop cleavable linkers to separate the coupled delivery molecules that have accomplished the guidance from the siRNA molecules.

### Summary

The embodiments of the present invention provide a compound that can be used to conjugate nucleic acid a liver-targeted nucleic acid conjugated ligand, as well as their use for the delivery of nucleic acid molecules and their use in the field of disease treatment.

According to the first aspect, the present invention provides a cleavable compound, as shown in formula (Z-1), or a pharmaceutically acceptable salt thereof,

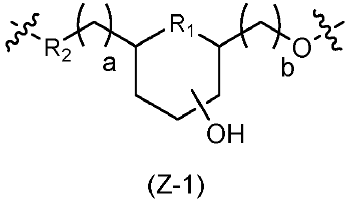

Where R₁ is O, S, NR₃, or CR₃R₄, wherein R₃ and R₄ are independent hydrogen, halogen substituted or unsubstituted aliphatic group, substituted or unsubstituted aryl group, substituted or unsubstituted heteroaryl group, substituted or unsubstituted heterocyclic group, or substituted or unsubstituted cycloalkyl group;

Where R₂ can be-O-, -S-, -NH-, -CH₂-, -C(O)-, -OC(O)-, -C(O)O-, -NHC(O)-, -C(O)NH-, -CH₂NH-, -CH₂O-, -NH-C(O)-CH₂-, -C(O)-CH₂-NH-, or -NH(CO)NH-, wherein the - CH₂- can also be optionally substituted with substituent groups selected from halogen, alkyl (optionally replaced with substituent groups of hydroxyl, amino, or halogen), alkoxy, or alkylamino; a and b can be the same or different, and are the integers respectively selected from 0 to 20, preferably from 1 to 10, and further preferably from 1 to 5.

In a preferred embodiment, the general formula of compound Z is as follows (Z-2):

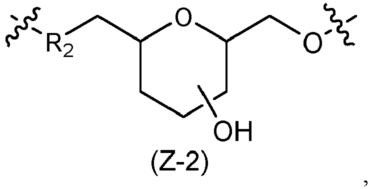

Preferably, R₂ is - NH -, and compound Z is:

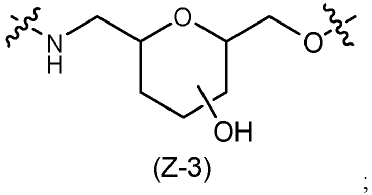

In another embodiment, R₂ is preferably -C(O)-, and compound Z is

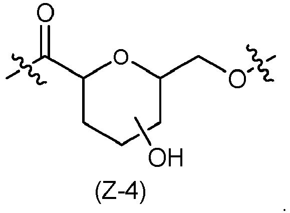

After delivering the nucleic acid sequence to the target location, the above compound breaks and only breaks at the R₂ position on the left and as a result, the delivered target product is predictable, avoiding the negative effects induced by irregular breaks.

According to the second aspect, the present invention provides a targeting ligand compound or a pharmaceutically acceptable salt thereof, including the aforementioned cleavable compound, with the general formula shown below (I): 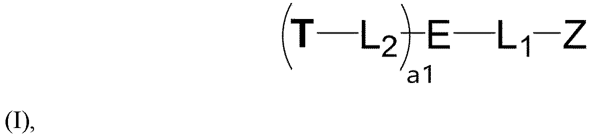
where Z is the cleavable compound shown in the above general formula (Z-1);
   or include the structure in general formula (II): 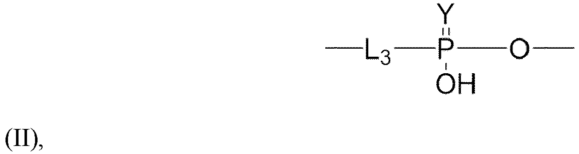
where L3 includes the cleavable compound shown in formula (Z-1) above.

Specifically, in general formula (I):
a1 is an integer from 1 to 10, preferably from 1 to 5, Z is the first linker of the nucleotide sequence (the above-mentioned cleavable compound Z-1), used to connect with the nucleotide sequence X, and the nucleotide sequence can be any nucleotide sequence, preferably an oligonucleotide sequence, and the nucleotide sequence can be a sense or antisense strand of siRNA, where Z and X can be directly connected or connected by chemical groups, and the general formula of Z is shown (Z-1) above, where one end of the O group is used to connect with the nucleotide sequence:

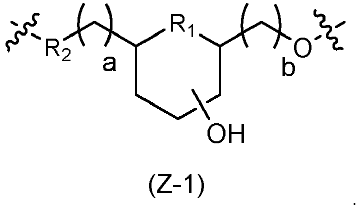

Preferably, the nucleotide sequence X includes no more than 30 nucleotides, and further preferably, no more than 23 nucleotides.

Specifically, in formula (I), L₁ is the second linker, E is the branch point group, both of which are directly connected with each other.

Preferably, the branch point group E is connected to a1 targeting ligand(s), wherein a1 is an integer from 0 to 10, preferably from 1 to 5; the targeting ligands includes a tethered portion L₂ and a targeting portion T in a 1:1 ratio.

In the above formula (II), conjugation with the nucleotide sequence can be the 5' end, or the 3' end, or both of the nucleotide sequence, and the conjugate with the nucleotide sequence X can be completed according to the following formula (III), where L₄ and L₃ can be the same or different, both are targeting portions, and Y is O, S, or N; b, c, d, and e are integers from 0 to 10.

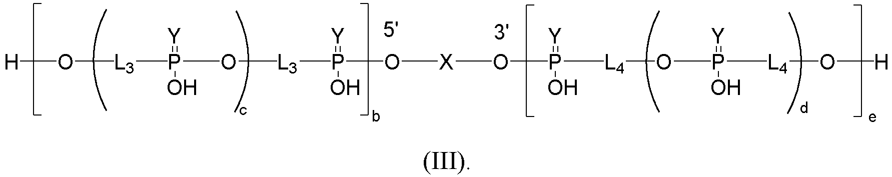

Specifically, in formula (II) and (III), L₃ includes a third linker, L₄ in formula (III) is the targeting ligand, and the targeting portion T in formula (I) may be the same as or different from L₃ and L₄ in formula (III). Preferably, L₃ and L₄ have the following structures, respectively: 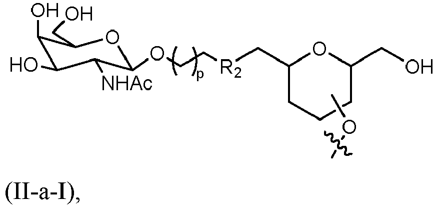 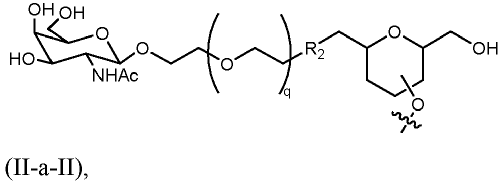 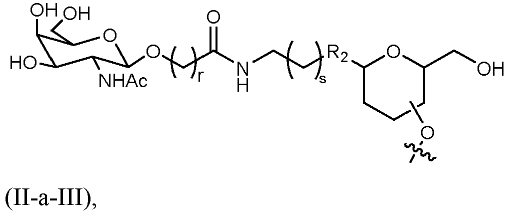 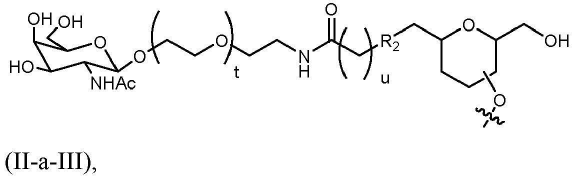
Where R₂ can be -O-, -S-, -NH-, -CH₂-, -C(O)-, -OC(O)-, -C(O)O-, -NHC(O)-, -C(O)NH-, -CH ₂ NH-, -CH₂O-, -NH-C(O)-CH₂-, -C(O)-CH₂-NH-, or -NH (CO) NH-, and -CH₂- can also be optionally replaced with substituent groups selected from halogens, alkyl groups (optionally further replaced with substituent groups selected from hydroxyl, amino, halogen, alkoxy, and alkylamino groups);
Wherein p, q, r, s, t, and u are integers independently selected from 0 to 20, preferably 1 to 10, and further preferably from 1 to 5.
In the said formula (III), X is the nucleotide sequence, and can be a sense or antisense strand;
Y is either O or S,
Wherein b, c, d, and e are integers from 0 to 10, respectively, preferably from 0 to 5, and b and e are not both 0. In one embodiment, b is 0 and e is an integer from 3 to 6. In a preferred embodiment, b=0, d=2, e=1.

Specifically, in the general formula (I), the second linker L₁ has the following structure:

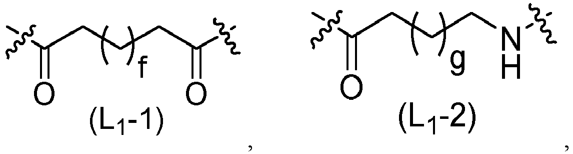

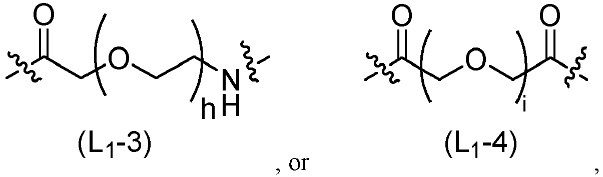

Where f, g, h, and i are integers from 1 to 20 respectively, preferably from 1 to 10, and further preferably from 1 to 5.

Specifically, in formula (I), the structural formula of compound L₂ is: 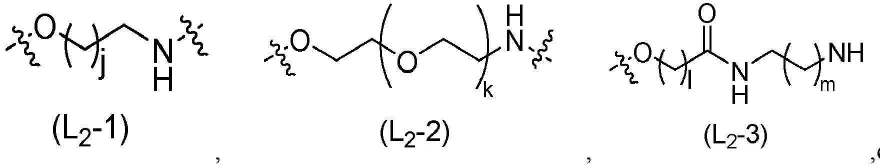 or

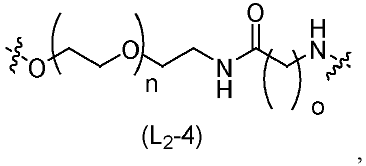

Where j, k, l, m, n, and o are integers from 1 to 20 respectively, preferably from 1 to 10, and further preferably from 1 to 5.

Further, the targeting portion T is selected from a tissue-specific targeting ligand, for example, a liver-specific targeting ligand or another tissue-specific targeting ligand; Preferably, the targeting portion T has a structure that can enhance the uptake of oligomers for target cells; In a preferred embodiment, the target cells are liver cells.

The oligonucleotide conjugate provided in the embodiments of the present invention can improve the efficiency for targeting portion to bind to cells or cell receptors, and enhance the working effect of RNA interference.

The embodiments of the present invention also specify the use of the compounds and pharmaceutically acceptable salts in formula (Z-1), (I), and (II) for delivering nucleic acid molecules. Specifically, the nucleic acid molecules can be delivered to in vivo specific cells.

In further, the compound or pharmaceutically acceptable salt thereof in formula (Z-1) breaks at R₂ after delivering the nucleic acid molecule to the target cell.

In further, the compound and pharmaceutically acceptable salt thereof in formula (I) and (II) are used for targeted binding to nucleic acids and delivery of nucleic acids to human or mammalian hepatocytes.

In further, the liver cells are liver parenchymal cells.

The compounds and pharmaceutically acceptable salts in formula (Z-1), (I), and (II) provided in the embodiments of the present invention are used as nucleic acid coupled delivery ligands or a portion thereof, and the delivery ligand can deliver target nucleic acids to designated cells with high delivery efficiency.

### Brief Description of the Drawings

FIG. 1A shows the detection results of a compound provided in one embodiment of the present application after 28-day inhibition of ANGPTL3 expression, and FIG. 1B shows the detection results after 35-day inhibition of ANGPTL3 expression;
FIG. 2 shows the detection results of LPA siRNA concentration in mouse liver and kidney in one embodiment of the present application.

### Detailed Description of the Embodiments

The present invention will be further detailed below in combination with embodiments and chemical reaction formulas, in order to elaborate the technical problems to be addressed by the present invention, the technical solutions, and beneficial effects more clearly. It should be appreciated that the specific embodiments described here are only intended to explain the present invention rather than to limit the present invention.

### Definition of Terms

The term "oligonucleotide" in this application, typically, refers to a polymer consisting of a plurality of nucleotide residues (deoxyribonucleotides or ribonucleotides, or their related structural variants or synthetic analogues) connected by phosphodiester bonds (or their related structural variants or synthetic analogues). Thus, although the term "oligonucleotide" generally refers to a nucleotide polymer in which the nucleotide residues and the linkages between them are naturally occurring nucleotide polymers, it should be understood that the term also includes various analogues, including but not limited to: peptide nucleic acid (PNA), amino phosphate ester, thiophosphate ester, methyl phosphonate, 2-O-methylribonucleic acid, etc. The exact size of the molecule depends on its specific application. Oligonucleotide is generally quite short, and usually has about 10-30 nucleotide residues, but this term can also refer to molecules of any length, although the terms "polynucleotide" or "nucleic acid" are generally used for larger oligonucleotides.

In some embodiments, oligonucleotides contain one or more unmodified ribonucleosides (RNA) and/or unmodified deoxyribonucleosides (DNA) and/or one or more modified nucleosides. The term "modified oligonucleotide" typically refers to the oligonucleotide containing at least one modified nucleoside and/or at least one modified nucleoside inter-molecular bond.

The term "modified nucleoside" in this application typically refers to, compared to naturally occurring RNA or DNA nucleoside, a nucleoside containing at least one chemical modification. Modified nucleosides include modified sugar moieties and/or modified nucleobases.

The term "nucleobase" in this application typically refers to a heterocyclic pyrimidine or purine compound, and is a component of all nucleic acids and includes adenine (A), guanine (G), cytosine (C), thymine (T), and uracil (U). Nucleotides can include modified nucleotides or nucleotide mimetics, abasic sites (Ab or X), or substitute parts. As used in this application, "nucleobase sequence" typically refers to the sequence of consecutive nucleobases that do not rely on any sugar, bonding, or nucleobase modification. The terms "unmodified nuclear base" or "naturally occurring nuclear base" usually refer to the naturally occurring heterocyclic nucleobases of RNA or DNA: purine bases adenine (A) and guanine (G); pyrimidine bases thymine (T), cytosine (C) (including 5-methylC), and uracil (U). "Modified nucleobase" usually refers to any nucleobase that is not naturally present.

The term "ligand" in this application typically refers to any compound or molecule that can covalently or otherwise chemically bind to a bioactive substance (such as oligonucleotides). In some embodiments, the ligand can directly or indirectly interact with another compound like a receptor, and the receptor with which the ligand interacts can exist on the cell surface or, alternatively, can be an intracellular and/or intercellular receptor. The interaction between the ligand and the receptor can lead to biochemical reactions, or can be merely a physical interaction or binding.

The present embodiment of the present application provides a cleavable compound as shown in formula (Z-1), 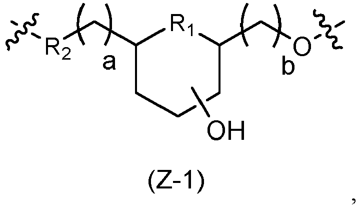
Where R₁ is O, S, NR₃, or CR₃R₄, where R₃ and R₄ are independent hydrogen, halogen, substituted or unsubstituted aliphatic group, substituted or unsubstituted aryl group, substituted or unsubstituted heteroaryl group, substituted or unsubstituted heterocyclic group, or substituted or unsubstituted cycloalkyl group;
Where R₂ can be -O-, -S-, -NH-, -CH₂-, -C(O)-, -OC(O)-, -C(O)O-, -NHC(O)-, -C(O)NH-, -CH₂NH-, -CH₂O-, -NH-C(O)-CH₂-, -C(O)-CH₂-NH-, or -NH(CO)NH-, and -CH₂- can also be optionally replaced with substituent groups of halogen, alkyl (optionally substituted from hydroxyl, amino, halogen substituents), alkoxy, or alkylamino; a and b can be the same or different, and are integers respectively selected from 0 to 20, preferably from 1 to 10, and further preferably from 1 to 5.

In a preferred embodiment, compound Z is: 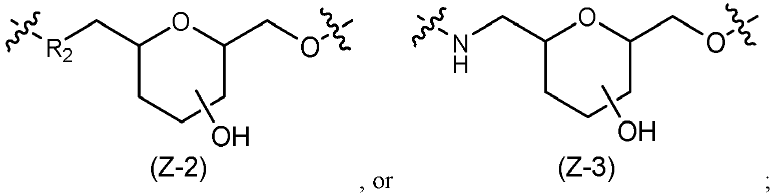 or

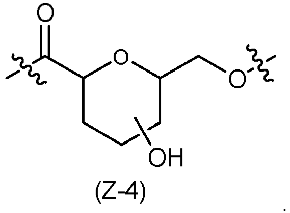

The O-group of the above compound binds to the nucleotide sequence, and breaks at the left R₂ after delivering the nucleic acid sequence to the target location.

The present embodiment also provides a liver-targeted ligand for oligonucleotides, with the general formula as follows, 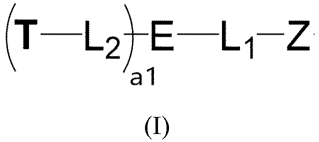 or 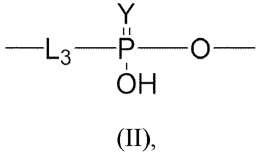 or with the following structure after binding to nucleotide sequence X:

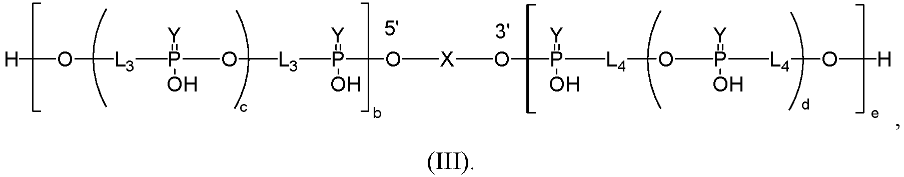

Specifically, in general formula (I):
X is the sense or antisense strand of a nucleotide, Z is the linker of sense or antisense strand, E is the branch point group, L₁ is the connection part between the linker of the sense or antisense strand and the branch point group E, T is the targeting portion, and L₂ is the tethered portion between the targeting portion and the branch point group E, wherein a1 is an integer selected from 1 to 10, preferably from 1 to 5, and further preferably 1 or 3.

In general formula (II) and (III):
L₃ and L₄ are targeting portions, X is the sense or antisense strand, Y is O, S, or N, where b, c, d, and e are integers selected from 0 to 10, preferably from 1 to 5.

Specifically, T, L₃, and L₄ mentioned above can be the same or different from each other.

In an embodiment, L₁ in the targeting ligand of formula (I) has the following general formula: 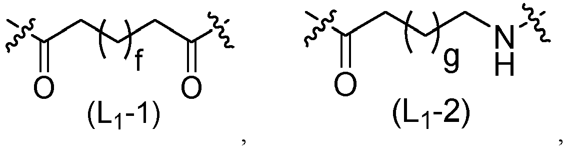 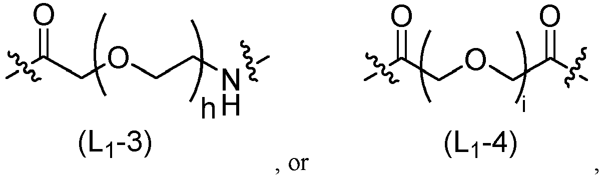 Where f, g, h, and i are integers from 1 to 20, preferably from 1 to 10, and further preferably from 1 to 5;

Preferably, L₁ has the following structure: 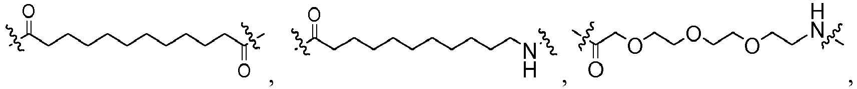 or

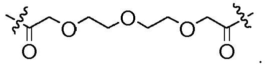

In an embodiment, in the general formula (I), the compound E is selected from:

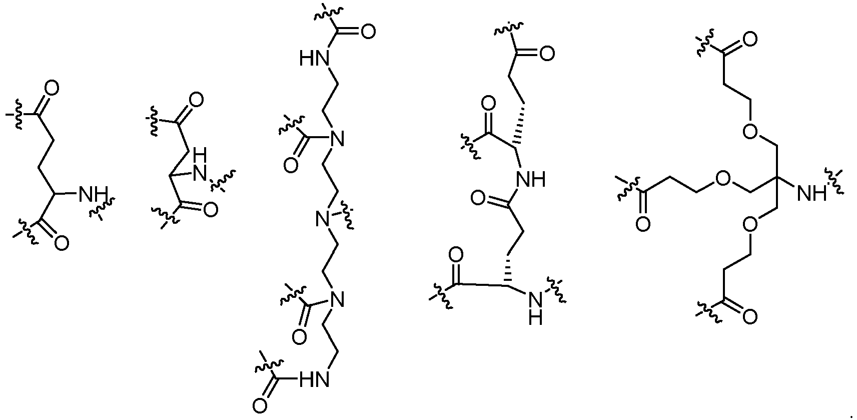

In an embodiment, compound L₂ in general formula (I) is 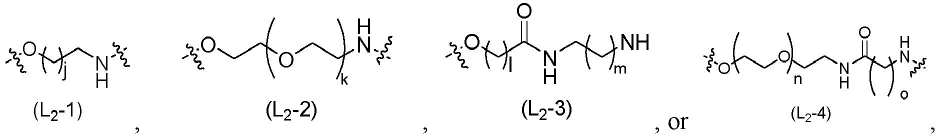 Where j, k, l, m, n, and o are integers from 1 to 20 respectively, preferably from 1 to 10, and further preferably from 1 to 5.

Preferably, L₂ has the following structural formula: 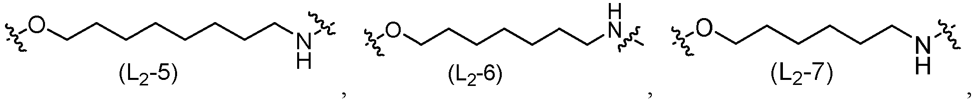 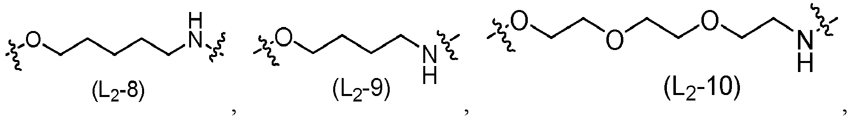 or

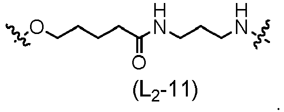

In the preferred embodiment, the general formula (I) has the following structure: 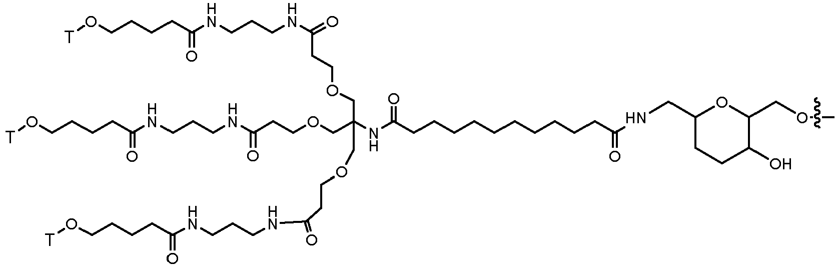
(I-I), where a1=3, 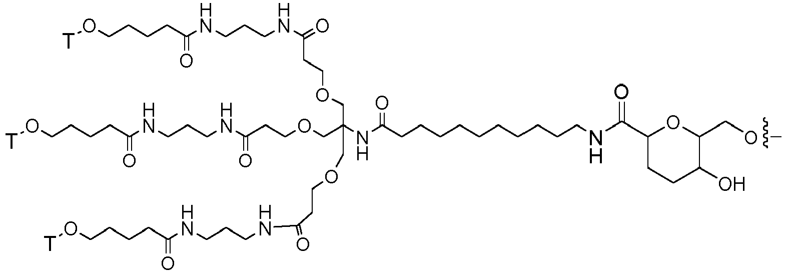
(I-II), where a1=3, 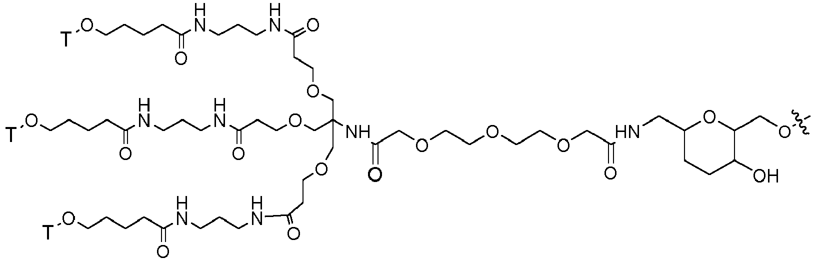
(I-III) , where a1=3, 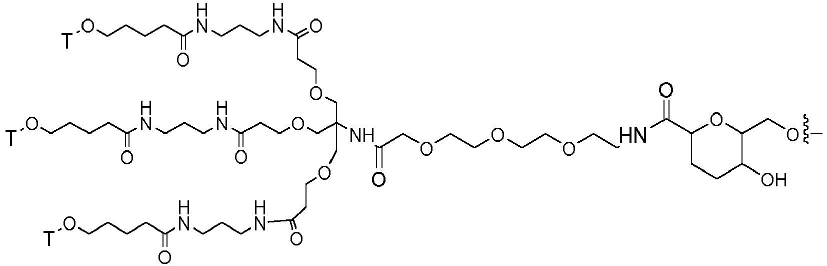
(I-IV) , where a1=3, 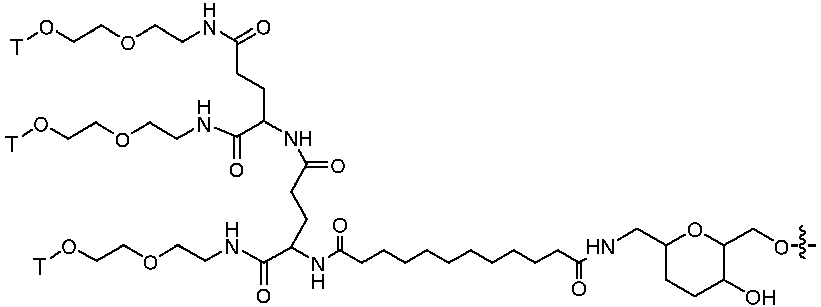
(I-V) , where a1=3, 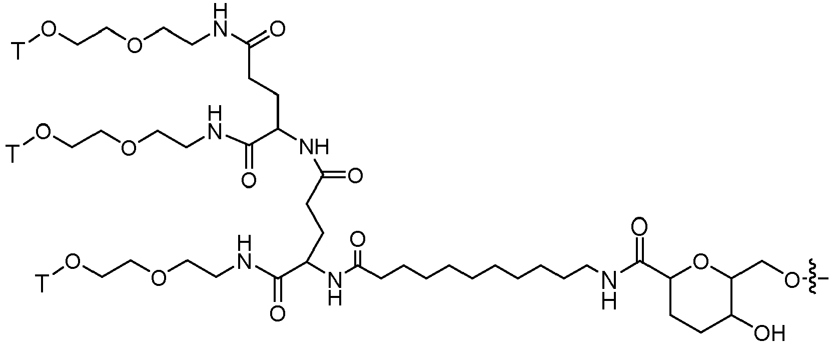
(I-VI), where a1=3, 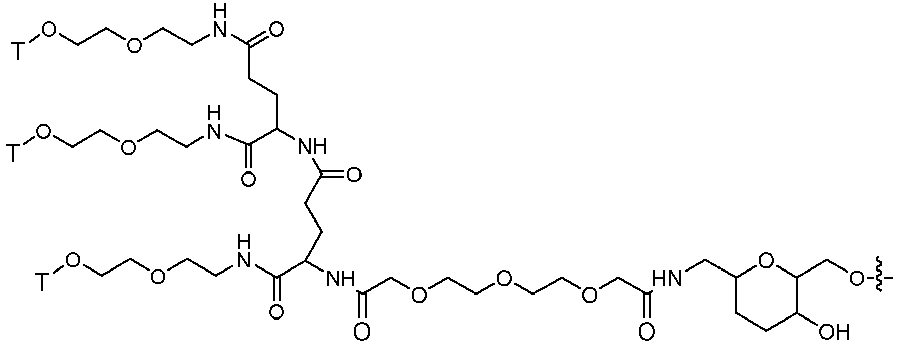
(I-VII) , where a1=3, or 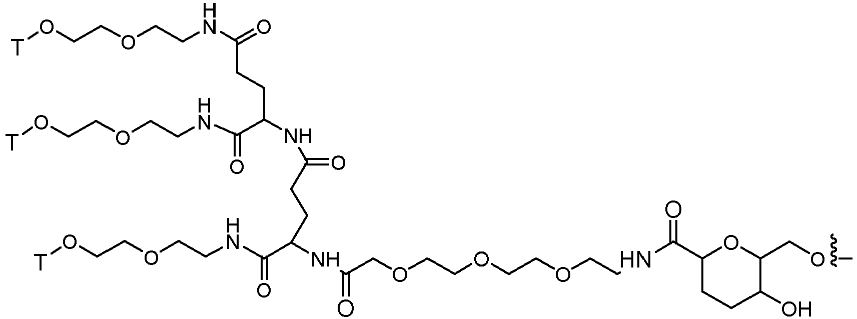
(I-VIII) , where a1=3.

In an embodiment, the targeting ligand T is selected from one of N-acetyl galactosamine, galactose, galactosamine, N-formyl galactosamine, N-propionyl galactosamine, N-butyryl galactosamine, and N-isobutyryl galactosamine, preferably N-acetyl galactosamine, with the structural formula as follows:

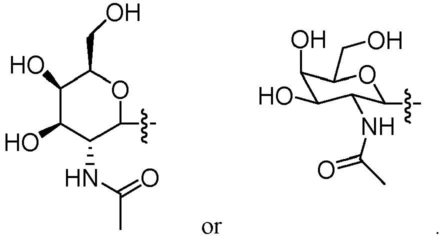

In a preferred embodiment, the targeting ligand shown in general formula (I) is connected to the end of siRNA via phosphate or thiophosphate or phosphonic acid groups.

In a preferred embodiment, the targeting ligand has the following structure: 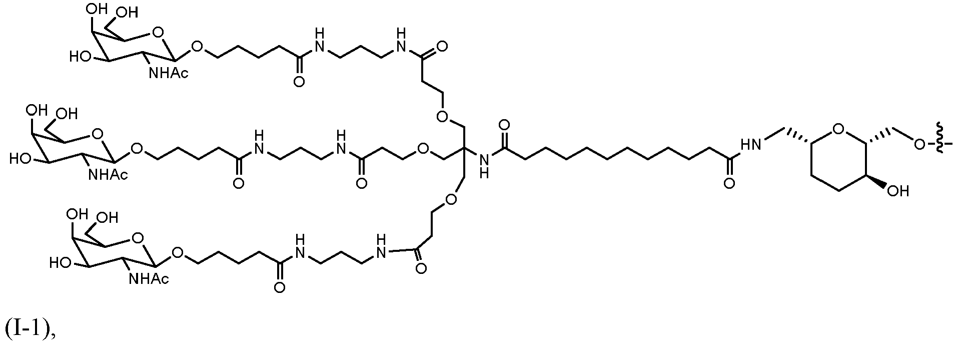 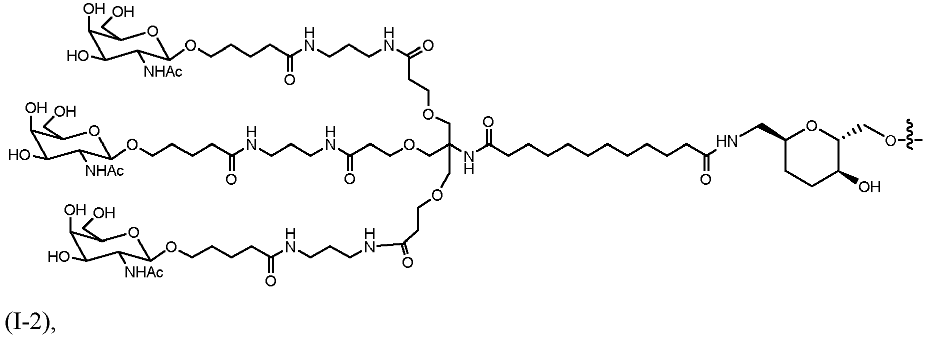 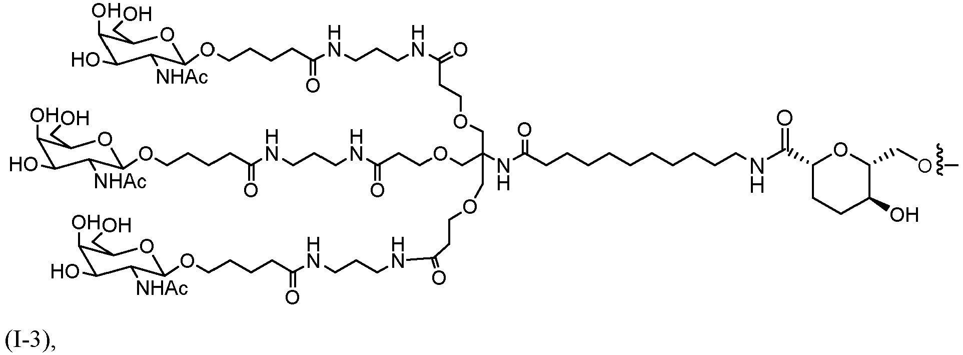 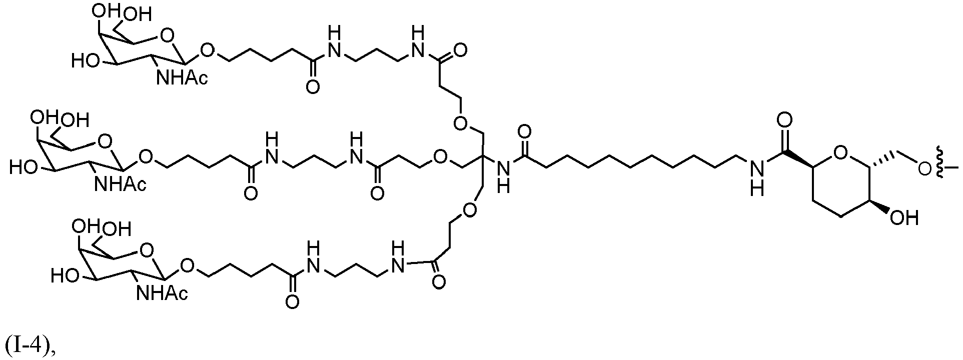 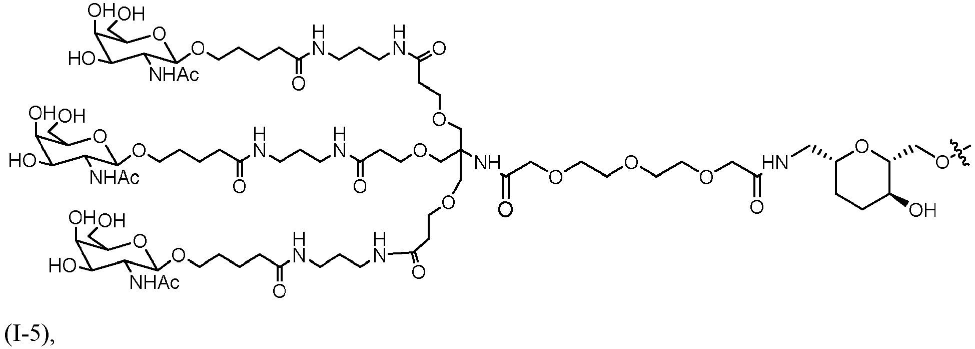 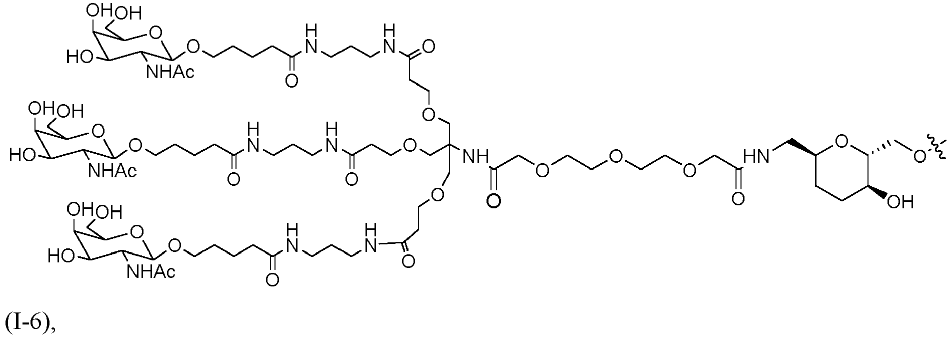 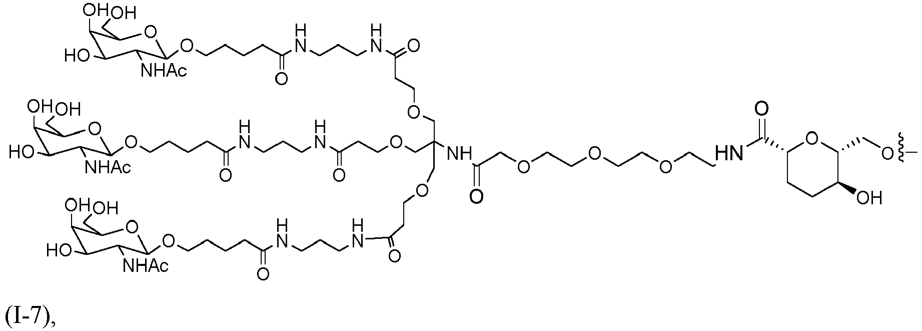 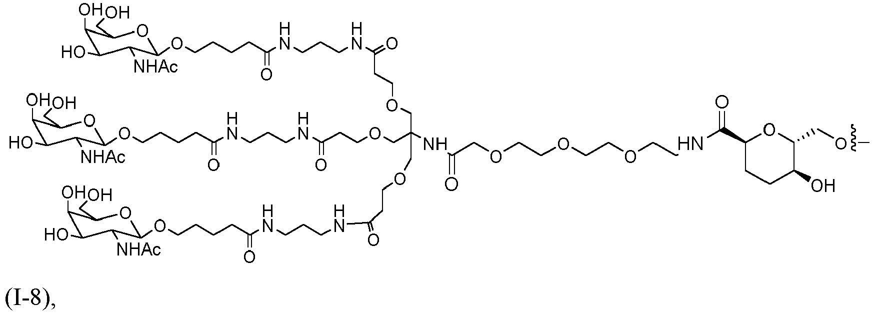 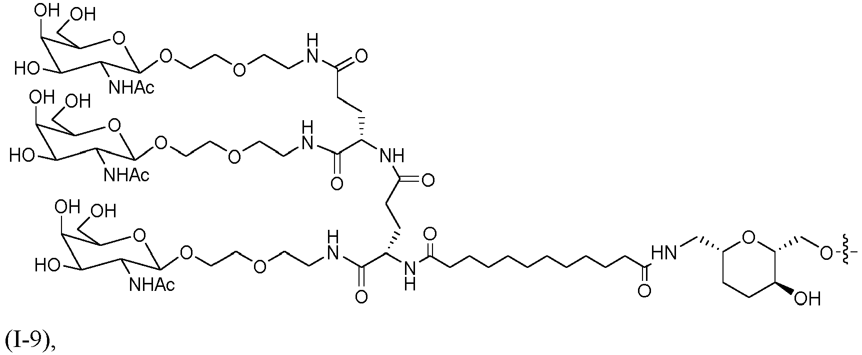 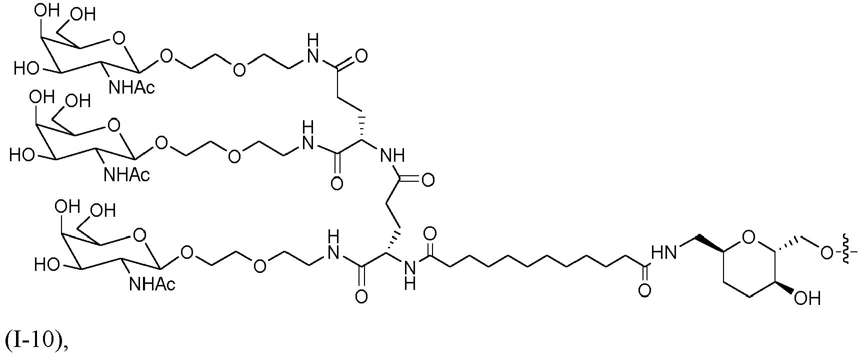 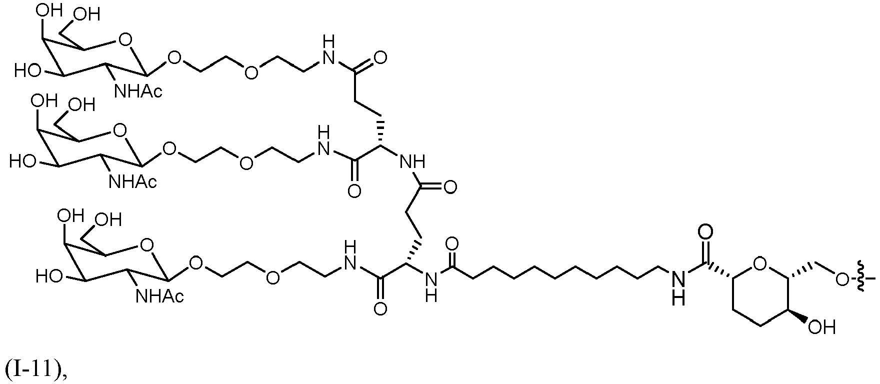 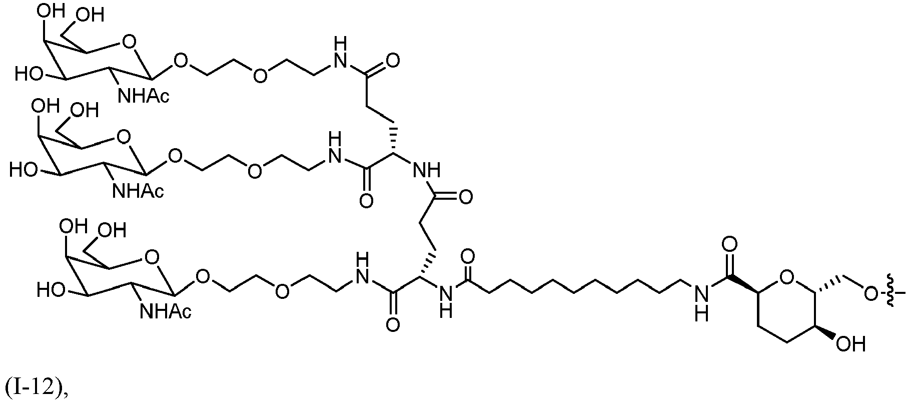 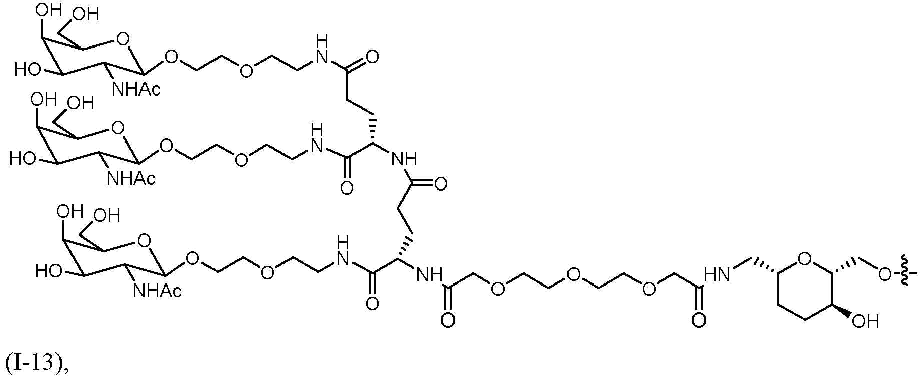 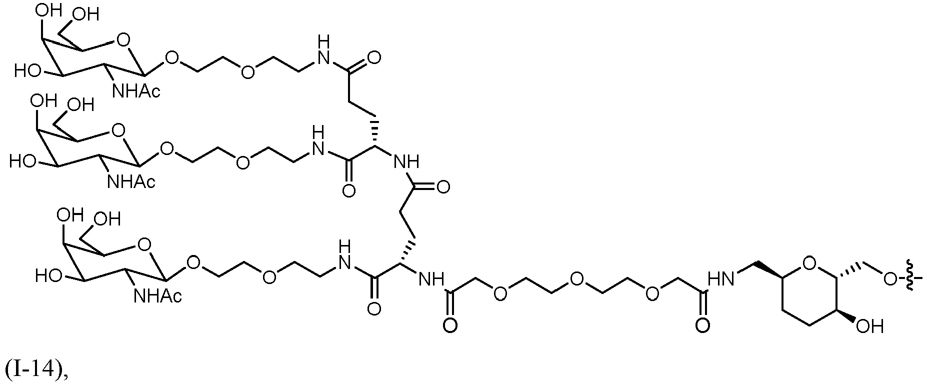 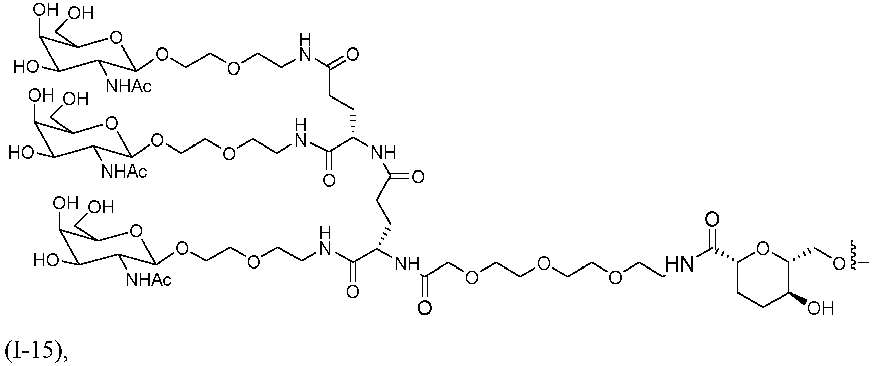 or

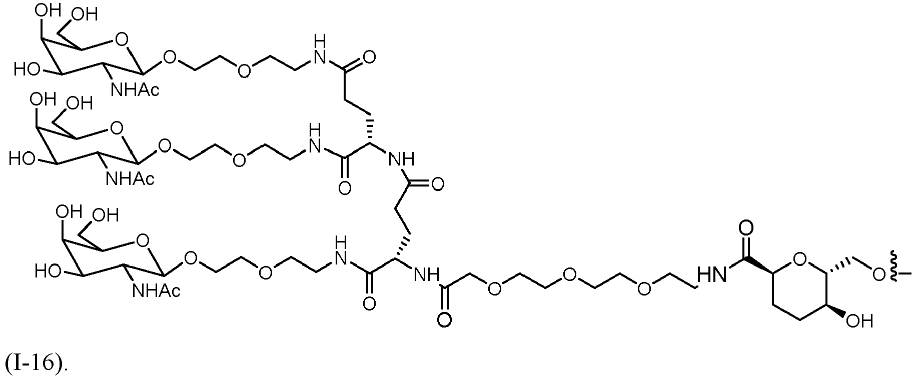

In an embodiment, the targeting ligand compound includes the following structures: 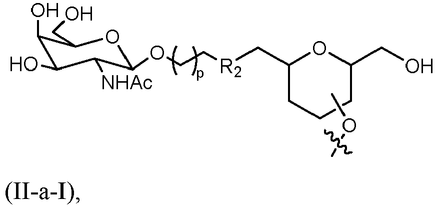 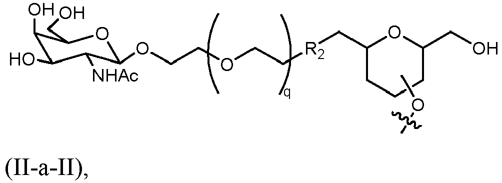 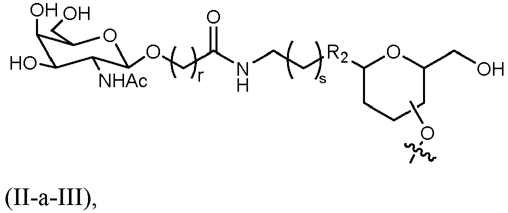 or 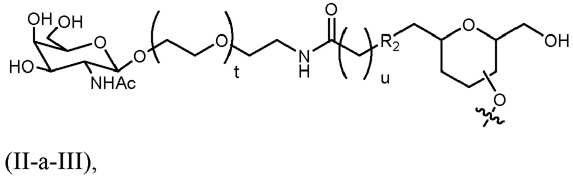 Where R₂ is -O-, -S-, -NH-, -CH₂-, -C(O)-, -OC(O)-, -C(O)O-, -NHC(O)-, -C(O)NH-, -CH ₂ NH-, -CH₂O-, -NH-C(O)-CH₂-, -C(O)-CH₂-NH- or -NH(CO)NH-, and -CH₂- can be optionally replaced with substituent groups of halogen, alkyl (optionally further replaced with substituent groups of hydroxyl, amino, halogen, alkoxy, and alkylamino), alkoxy, and alkylamino;

The p, q, r, s, t, and u are integers independently selected from 0 to 20, preferably from 1 to 10, and further preferably from 1 to 5.

In a preferred embodiment, the targeting ligand compound includes the following structures: 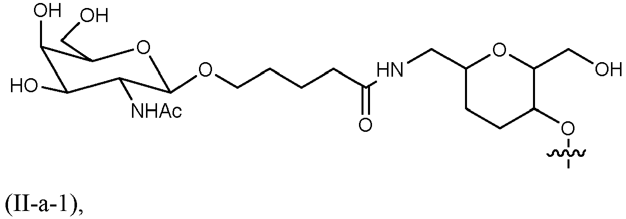 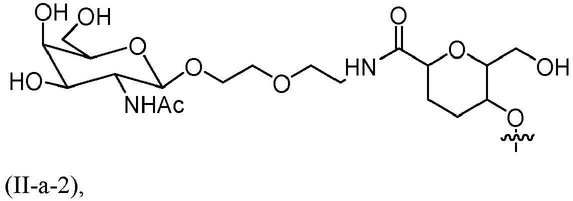 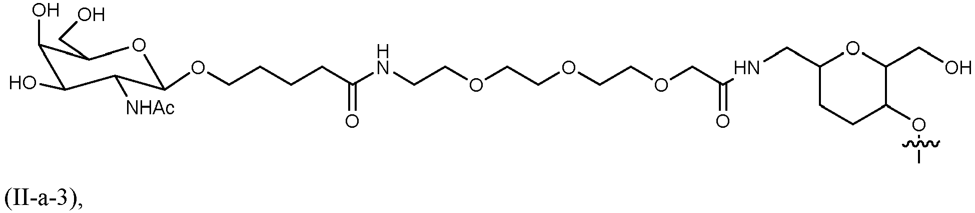 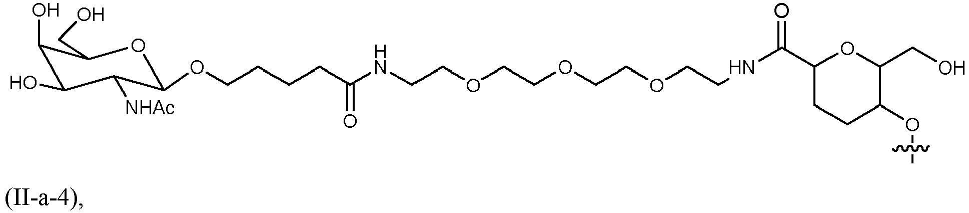 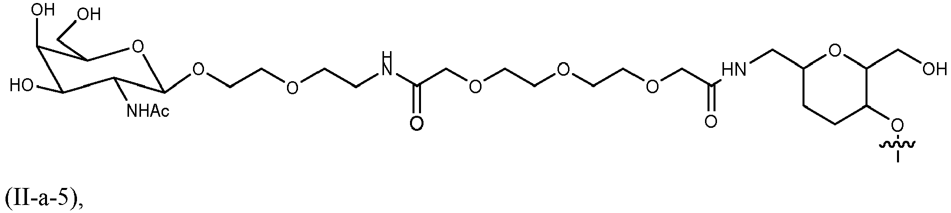 or

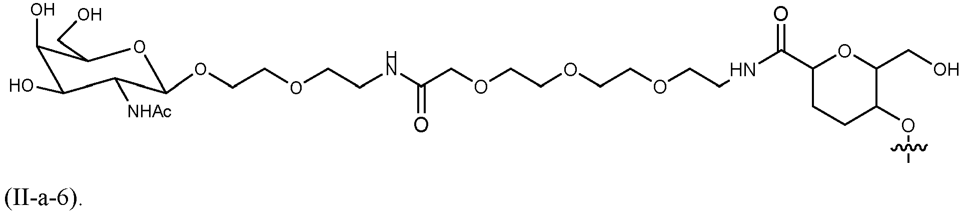

In another preferred embodiment, the targeting ligand compound includes the following structure: 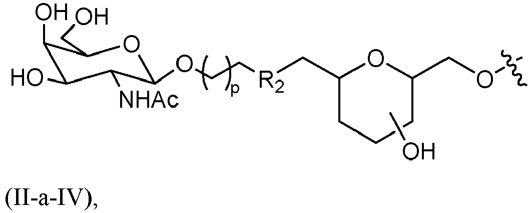 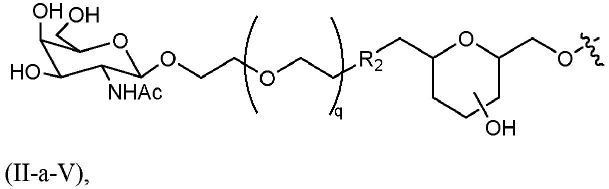 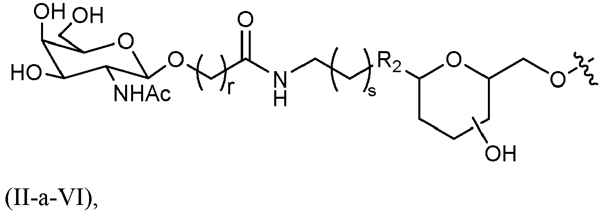 or 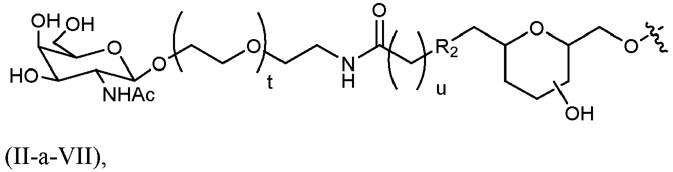
Where R₂ is -O-, -S-, -NH-, -CH₂-, -C(O)-, -OC(O)-, -C(O)O-, -NHC(O)-, -C(O)NH-, -CH₂NH-, -CH₂O-, -NH-C(O)-CH₂-, -C(O)-CH₂-NH- or -NH(CO)NH-, and -CH₂- can be optionally replaced with substituent groups of halogen, alkyl (optionally further replaced with substituent groups of hydroxyl, amino, halogen, alkoxy, and alkylamino), alkoxy, and alkylamino;
Wherein p, q, r, s, t, and u are integers independently selected from 0 to 20, preferably from 1 to 10, and further preferably from 1 to 5. In a preferred embodiment, the targeting ligand compound includes the following structure: 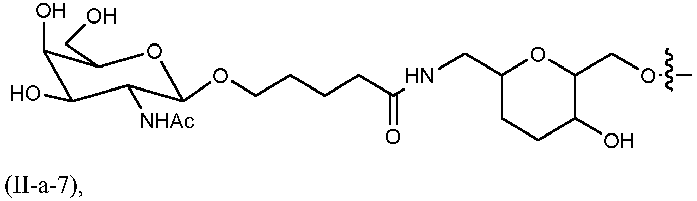 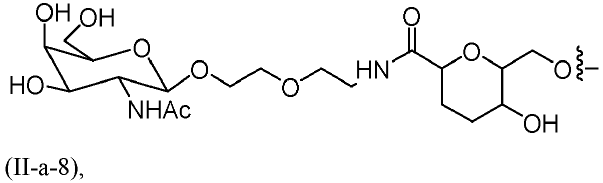 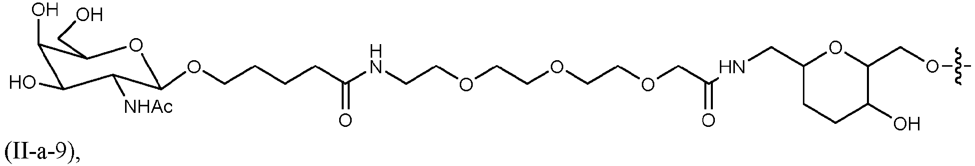 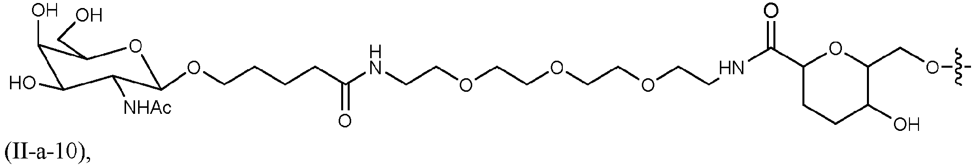 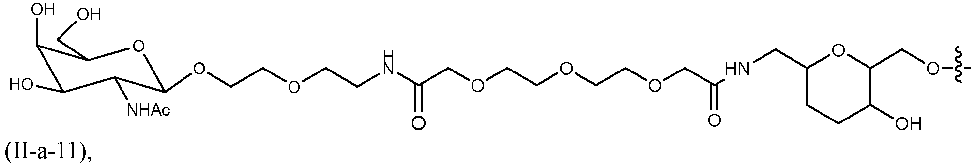 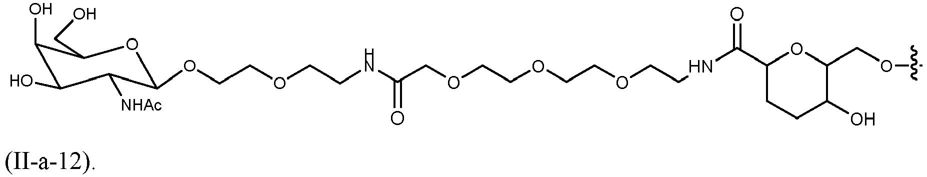 In a preferred embodiment, the general formula (III) has the following structure: 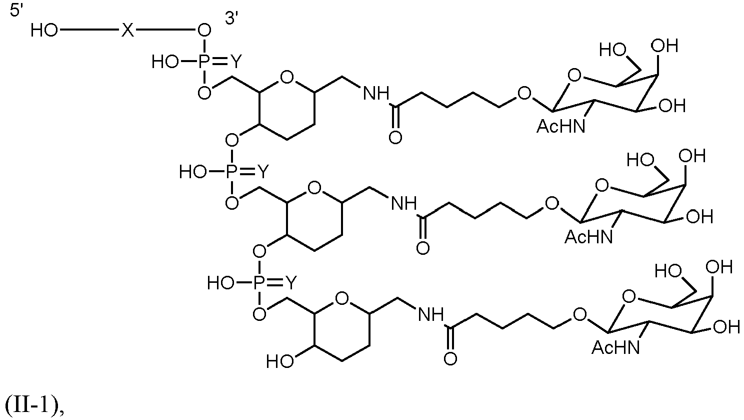 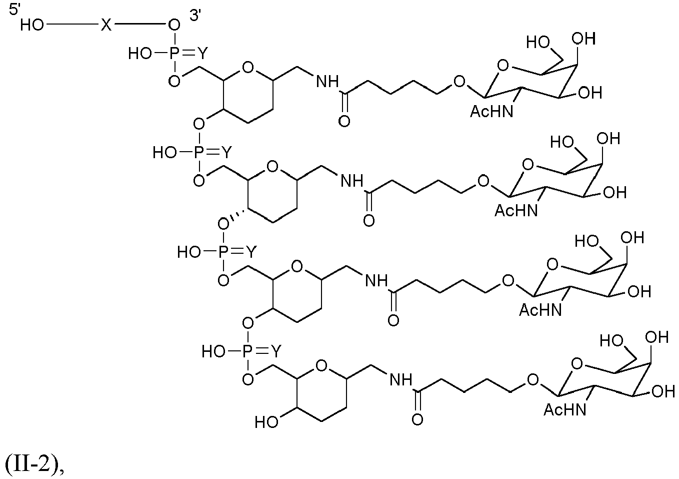 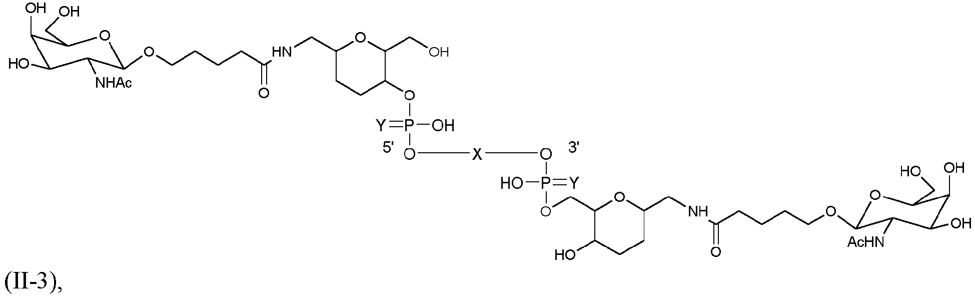 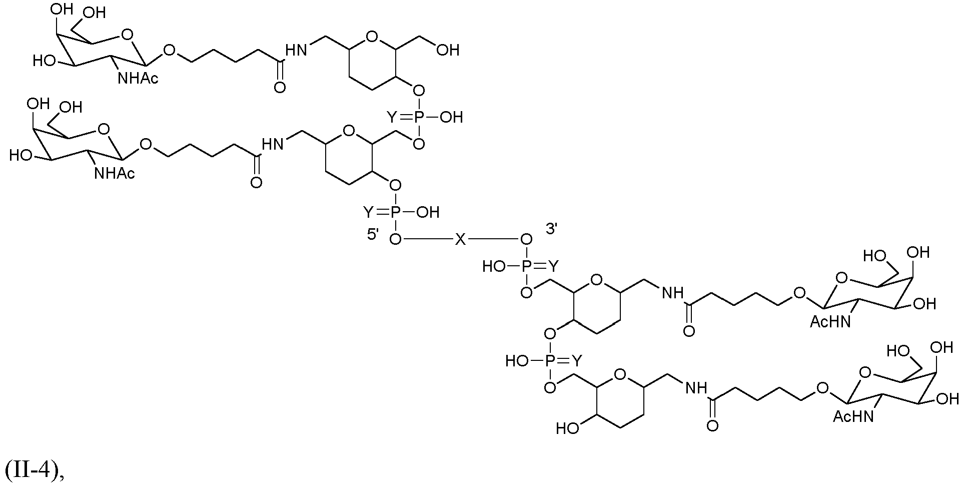 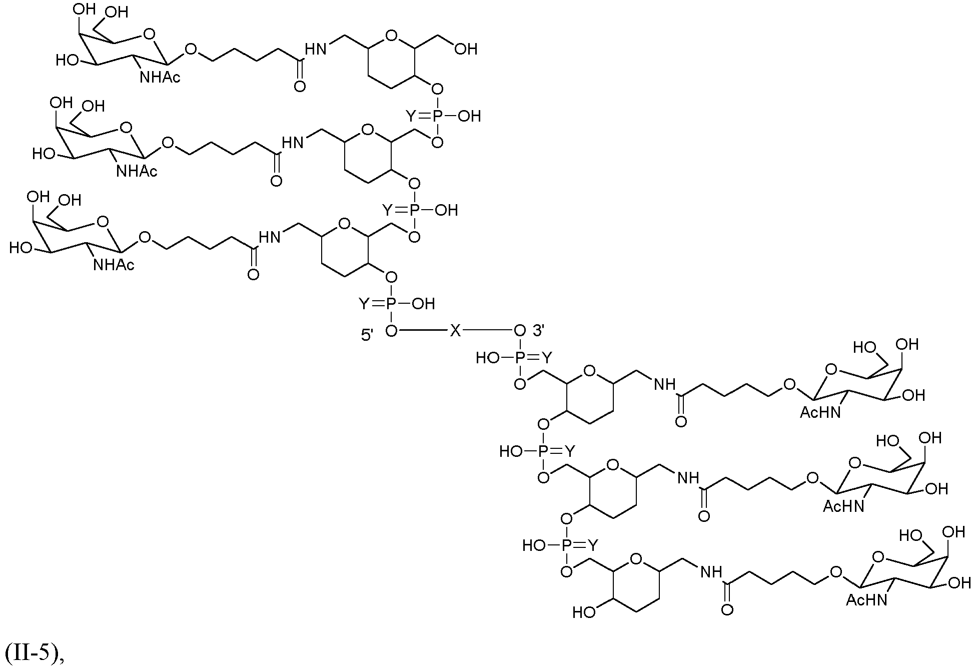 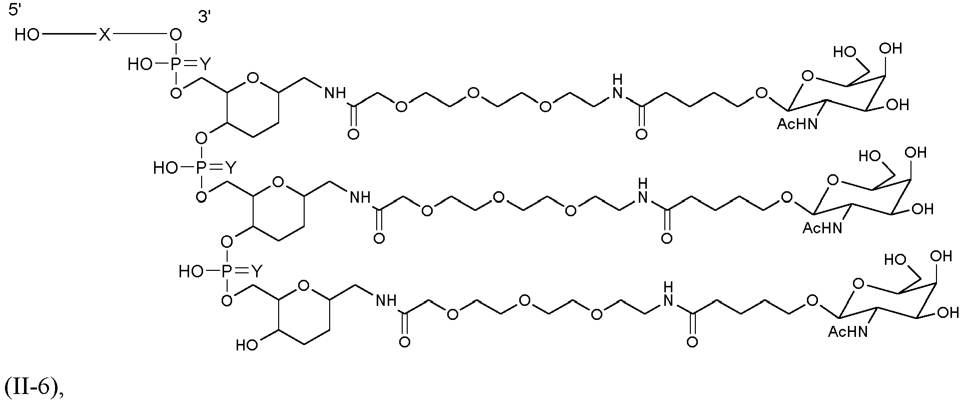 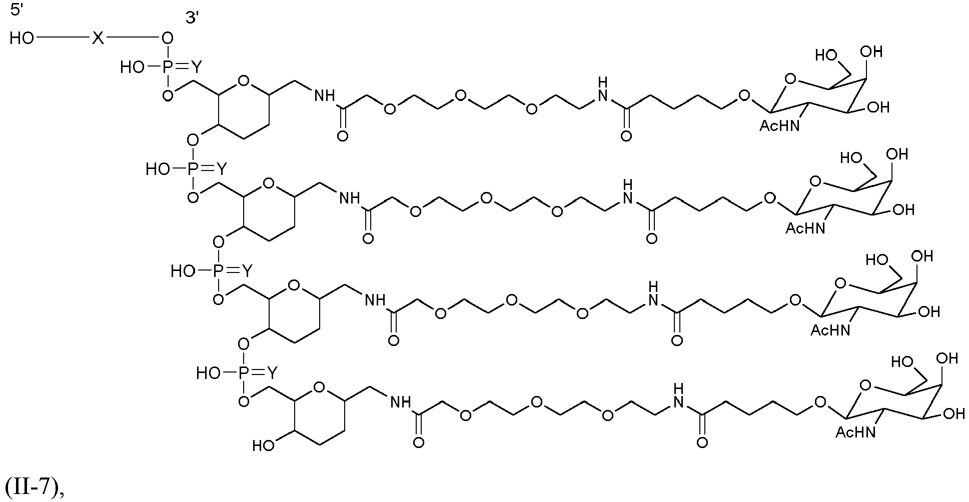 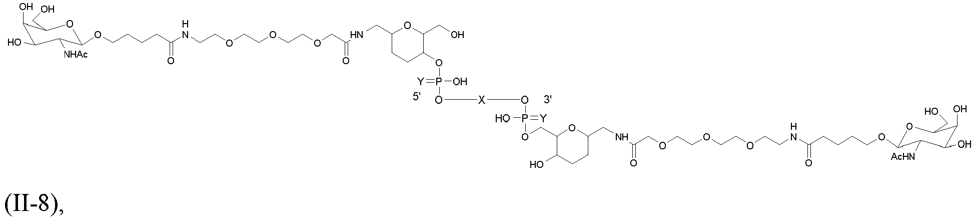 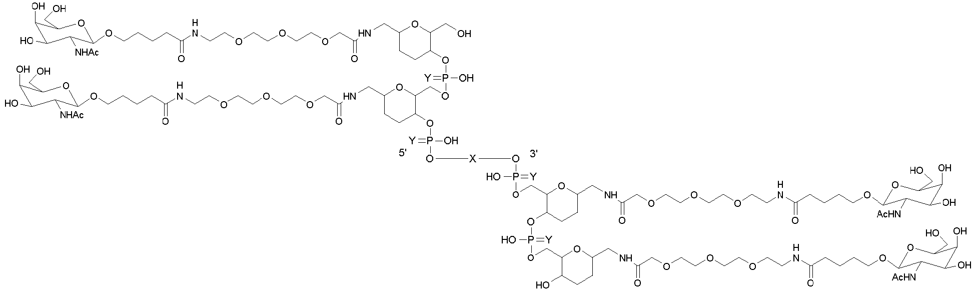 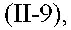 or 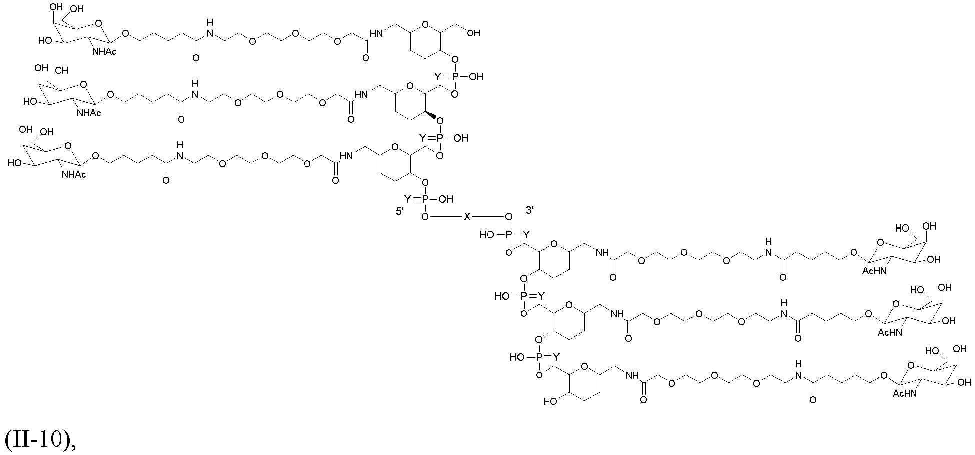
Where Y is either O or S.

In another preferred embodiment, the targeting ligand compound shown in formula (III) that binds to nucleotide X has the following structure: 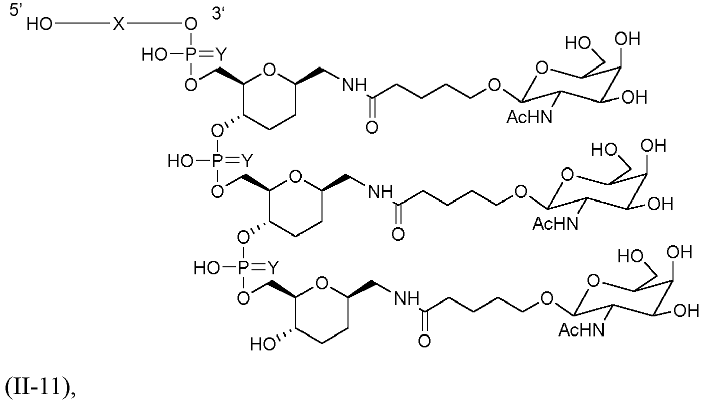 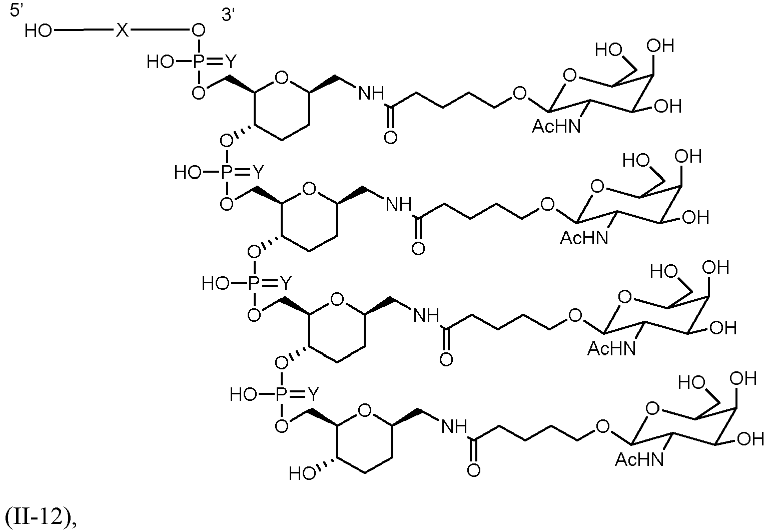 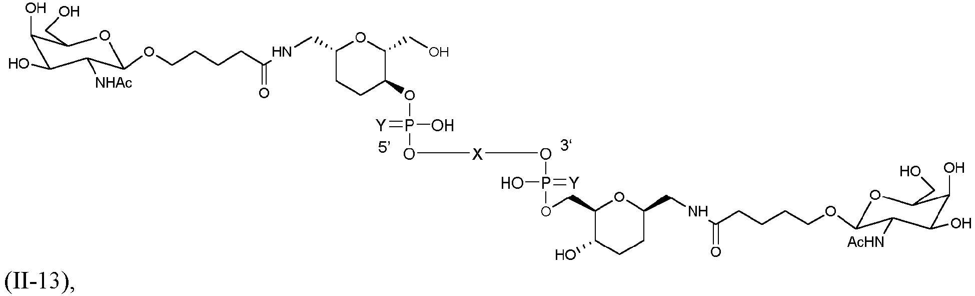 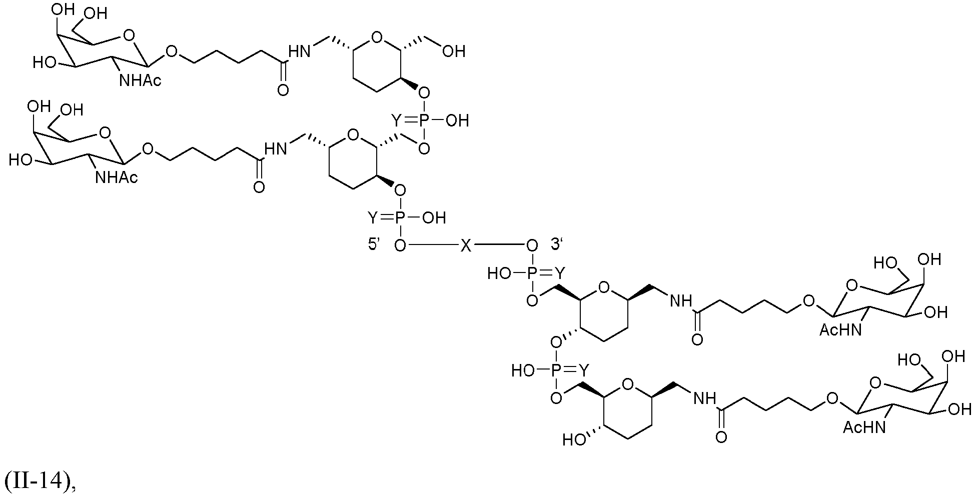 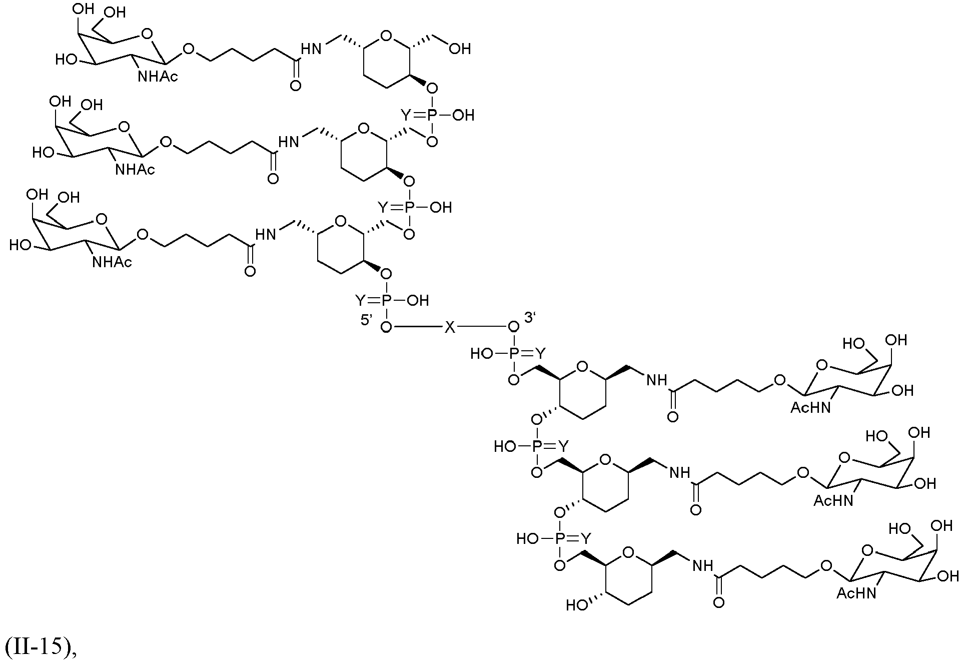 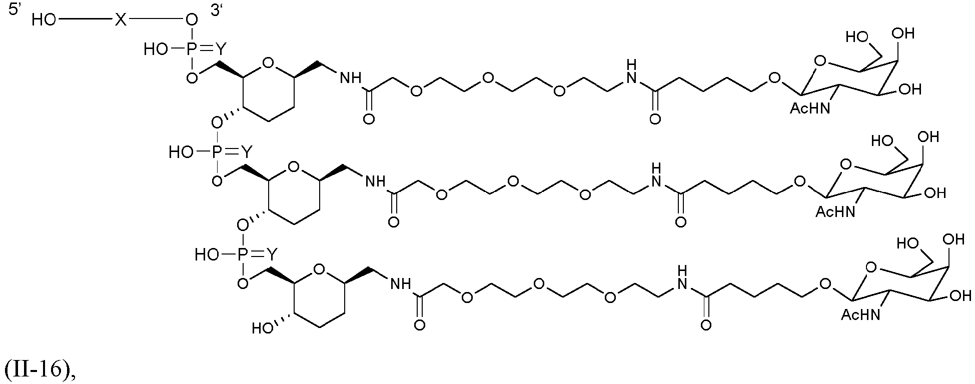 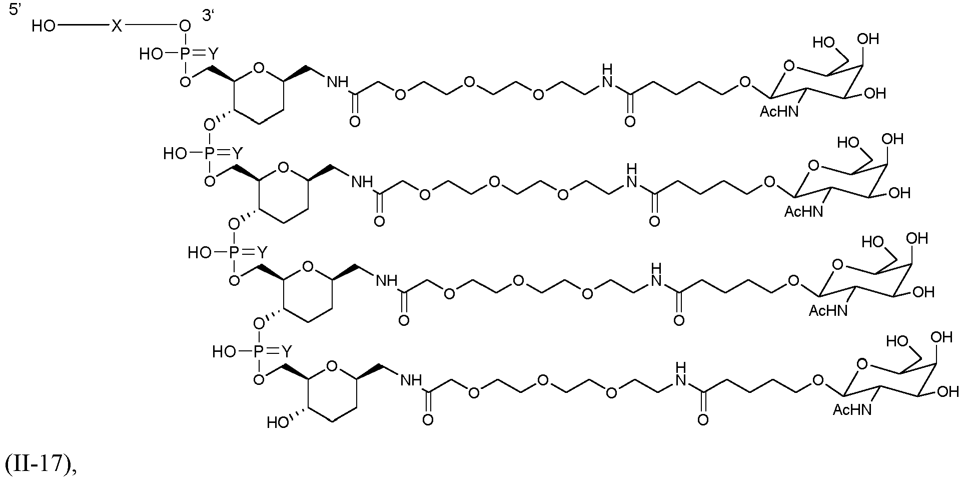 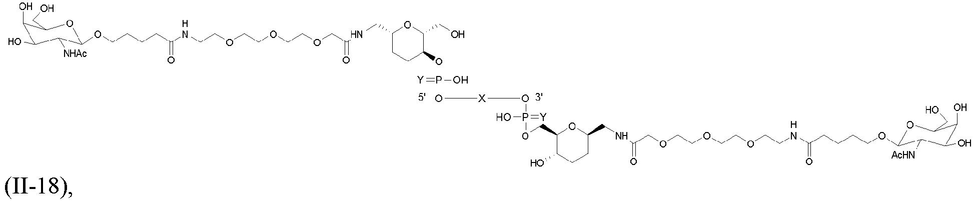 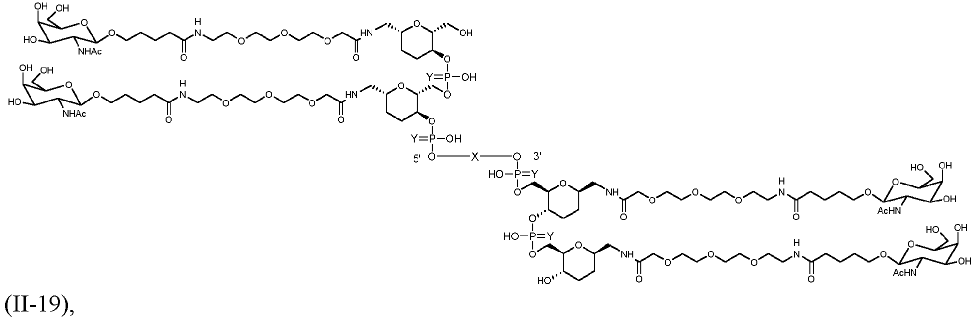 or 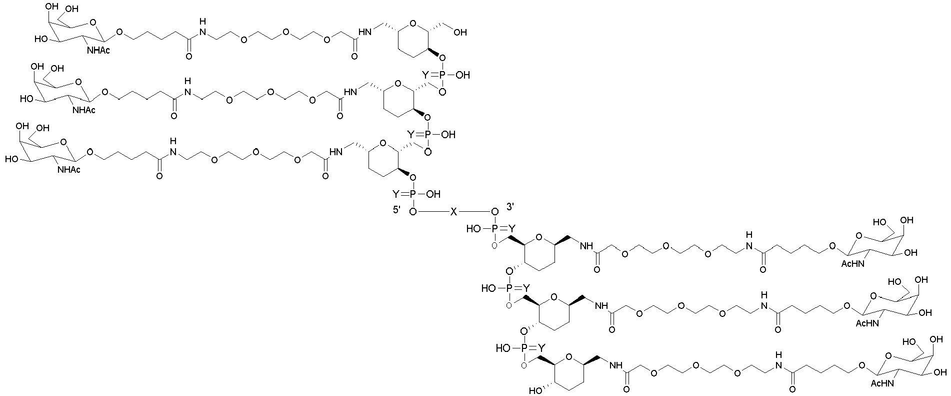 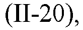 Where Y is either O or S or N.

In some embodiments, the sense strand can be selected from the nucleotide sequence shown in the following formula:
5'-NmsNmsNmNmNmNmNmNmNfNfNfNmNmNmNmNmNmNmNmNmNm-3', or,
5'-NmsNmsNmNmNmNmNmNmNfNfNfNmNmNmNmNmNmNmNmNmNms-3, or,
5'-NmNmNmNmNmNmNmNmNfNfNfNmNmNmNmNmNmNmNmNmNm-3', or,
5'-sNmNmNmNmNmNmNmNmNfNfNfNmNmNmNmNmNmNmNmNmNm-3' or,
5'-sNmNmNmNmNmNmNmNmNfNfNfNmNmNmNmNmNmNmNmNmNms-3',

Where Nm represents any methoxy modified nucleotides, such as 2'-O-methyl modified cytosine (C), guanine (G), uracil (U), adenine (A), thymine (T); Nf represents any 2'-fluoro modified nucleotides, such as fluoro modified C, G, U, A, and T;

The lowercase letter s in the middle of the uppercase letters indicates that the two nucleotides adjacent to the letter s are connected by a thiophosphate group. The lowercase letter s at the first 3 'end indicates that the nucleotide to the left of the letter s is connected by a thiophosphate group, and the lowercase letter s at the first 5' end indicates that the nucleotide to the right of the letter s is connected by a thiophosphate group.

In some embodiments, the antisense strand has a nucleotide sequence as shown in the following formula: 
5’-Nms’Nfs’Nm’Nm’Nm’Nf’Nm’Nm’Nm’Nm’Nm’Nm’Nm’Nf’Nm’Nf’Nm’Nm’Nm’Nm’Nms’
Nms’Nm -3’,
 Where Nm' represents any methoxy modified nucleotides, such as methoxy modified C, G, U, A, and T; Nf represents any fluoro modified nucleotides, such as fluoro modified C, G, U, A, and T;

The lowercase letter s in the middle of the uppercase letter indicates that the two nucleotides adjacent to the letter s are connected by thiophosphate groups.

The synthetic route of targeting ligand of present application is described below through specific embodiments.

### Example 1: Synthesis of amino galactose compound GENO-Gal-5 connected to a solid-phase carrier

### (1) Synthetic route

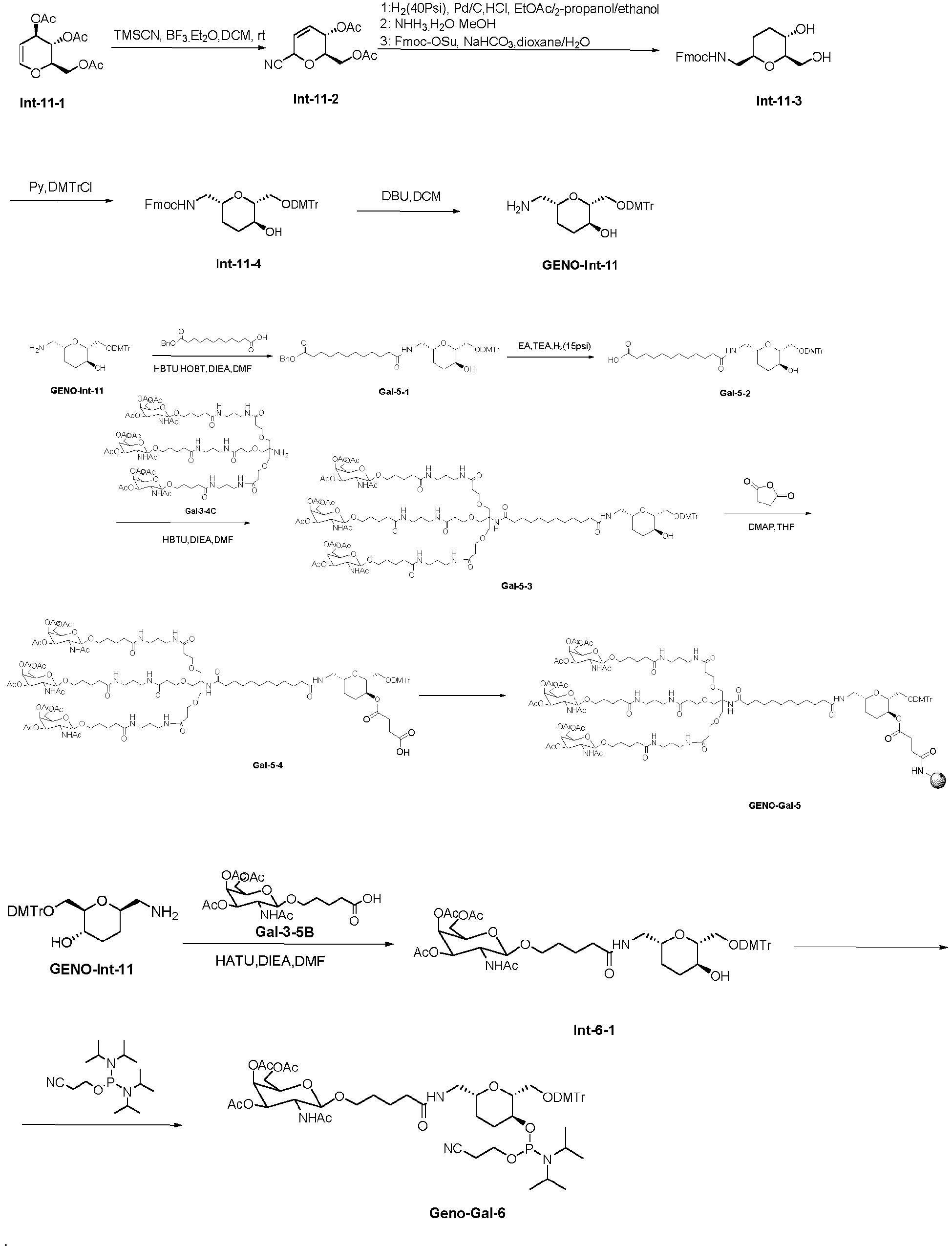

### (2) Specific synthesis steps

### Preparation of compound Int-11-2

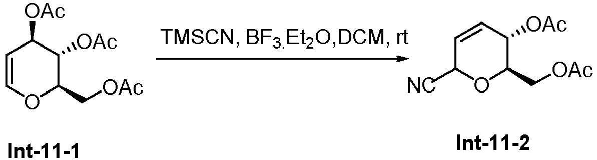

Int-11-1 (10 g) was dissolved in (140 mL) DCM (dichloromethane) and cooled to 0 °C. TMSCN (Trimethylsilyl cyanide) (4.01 g) and BF₃ · Et₂O (2.83 mL) were added dropwise, and reacting for 10 minutes. The reaction was monitor using TLC plate (using Hexane: EtOAc=5:1, KMnO₄ coloration, raw material Rf=0.3, α configuration Rf=0.28, β configuration Rf=0.27), and the raw material was completely reacted. After the reaction was complete, 100 mL of saturated NaHCO₃ aqueous solution was added to the reaction solution, and another 100 mL DCM was added to separate the organic phase. Washed the organic phase with saturated salt water once to concentrate the organic phase, then dissolved it in EtOAc (200 mL), washed with NaHCO₃ aqueous solution (100 mL) once and saturated salt water once, dried the organic phase with anhydrous sodium sulfate, and then concentrated the organic phase. Then, a positive silica gel column was used for purification and slowly increased the polarity. α configuration appeared when Hexane/EtOAc=20%, and β configuration appeared at 25%. α configuration produced a (5.3 g) white solid, while β configuration produced a (3.7 g) colorless oil like substance.

¹H NMR (400 MHz, DMSO) *δ* 6.03~6.06 (dt, 1H), 5.91-5.94 (dt, 1H ), 5.35 (dq, 1H), 5.12 (m, 1H), 4.30 (dd, 1H), 4.30 (dd, 1H), 3.82 (ddd, 1H), 2.10-2.12 (2s, 6H).

### 2) Preparation of compound Int-11-3:

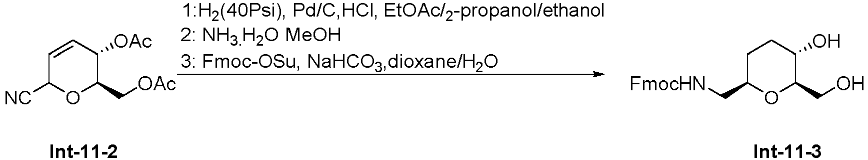

HCl aqueous solution (1M, 17.2 mL) was added to a suspension of Int-11-2 (3.7g, β configuration, colorless oil) and 10% Pd/C (377 mg), and the mixture of ethyl acetate/2-propanol/ethanol (2:1:1, 90 mL in total) was added, and the reaction was stirred under hydrogen gas (40 Psi) for 48 hours. Monitored the reaction using TLC, removed the catalyst through diatomaceous earth filtration, and concentrate the solvent under reduced pressure. Take the crude product twice with toluene, then dissolved in 10mL methanol, added 28% NH₃ · H₂O(30 mL), stirred at room temperature for 16 hours, and after the concentrate of reaction solution, and take reaction solution for three times with a 1:1 mixture of toluene and acetonitrile (50 mL). Dissolved 1 g of above crude product in 40 mL of H₂O and cooled to 0 °C, added NaHCO₃ (1.4 g) and Na₂CO₃ (0.88 g), dissolved FmocOSu (2.13 g) in dioxane (40 mL) solvent, then added dropwise to the above aqueous solution and react at room temperature for 1 hour. Monitor reaction using TLC (DCM: MeOH=10: 1, 254nm, Rf=0.5). Purification by positive column with MeOH/DCM=5% product, and obtain Int-11-3 (340 mg) colorless solid.

¹H NMR (CDCl₃): *δ* 7.76-7.78 (d, 2H), 7.58-7.60 (d, 2H), 7.39-7.42 (t, 2H), 7.30-7.34 (t, 2H), 5.12 (t, 1H), 4.42-4.52 (m, 2H), 4.22 (t, 1H), 3.84 (br. s, 2H). 3.47-3.57 (m, 3H), 3.11-3.24 (m, 2H), 1.30-1.56, 1.67-1.72, 2.05-2.22 (m, 4H).

### 3) Preparation of compound Int-11-4

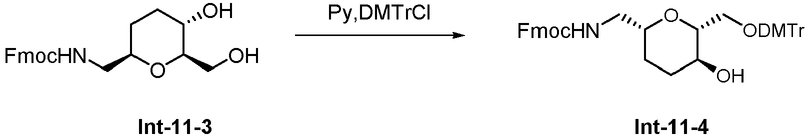

Dissolved Int-11-3 (340 mg) in 2 ml of pyridine and cooled to 0 °C. After dissolving DMTrCl (450 mg) in 2 ml of pyridine, it was added dropwise to the above solution. Monitor using TLC and the raw material was reacted completely. Added 5 ml of aqueous solution to quench the reaction, and purified by reverse column C18 with MeCN/ H₂O=80%, and the reaction product was int-11-4 (290 mg).

### Preparation of compound GENO-Int-11

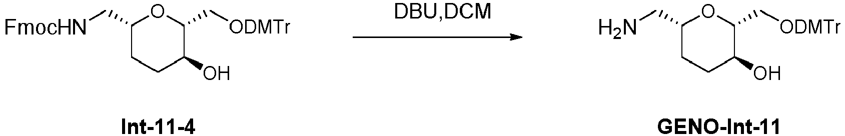

Dissolved Int-11-4 (700 mg, 1.02 mmol) in 2 mL of dichloromethane, then added DBU (310 mg, 2.04 mmol) to the above solution, monitor using TLC, and the raw material was reacted completely. Added 5 ml of saturated Na₂CO₃ solution to quench the reaction, extracted with 20 mL of dichloromethane, concentrate the organic phase with DCM: MeOH=10:1, (254 nm, Rf=0.5). Purified by positive column, MeOH/DCM=5%, and obtain yellow solid GENO-Int-11 (550 mg).

### Preparation of compound Gal-5-1

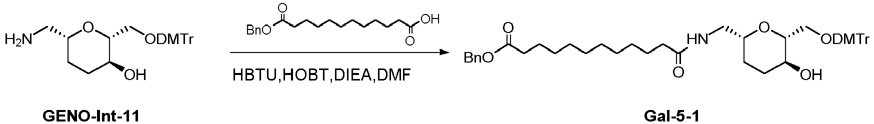

Dissolved 1,12-dodecanedioic acid monobenzyl ester (387 mg, 1.2 mmol) in anhydrous DMF (N, N-Dimethylformamide) (10 mL) solvent, added HBTU (O-Benzotriazole-N, N, N ', N' - tetramethyluronium hexafluorophosphate) (546 mg, 1.44 mmol), DIEA (N, N-Diisopropyl amine) (0.65 mL, 3.6 mmol), HOBT (1-Hydroxybenzotriazole) (324 mg, 2.4 mmol) mmol and GENO-Int-11 (560 mg, 1.2 mmol), then stirred overnight under argon protection at room temperature. Purified the reaction solution by reverse column C18, MeCN/H₂O=70%, and obtained the product Gal-5-1 (200 mg).

¹H NMR (400 MHz, DMSO) *δ* 7.68 (t, 1H), 7.49 - 7.12 (m, 14H), 6.86 (d, 4H), 5.07 (s, 2H), 4.58 (d, 1H), 3.73 (s, 6H), 3.38 (dd, 1H), 3.28 - 3.16 (m, 3H), 3.22-2.85 (m, 3H), 2.41 - 2.25 (m, 2H), 2.11 -2.00 (m, 2H), 1.92 (dd, 1H), 1.64 (d, 1H), 1.57 - 1.41 (m, 4H), 1.27 - 1.06 (m, 14H).

### Preparation of compound Gal-5-2

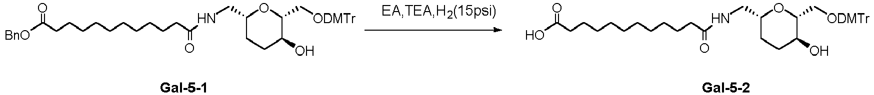

Dissolved Gal-5-1 (200 mg, 0.26 mmol) in 5 mL of ethyl acetate, added 50 mg of palladium charcoal and triethylamine (0.11 mL, 0.78 mmol), and stirred the reaction solution in hydrogen gas (15 psi) at room temperature for 16 hours. Filterred and concentrate the reaction solution to obtain Gal-5-2 (150 mg).

### Preparation of compound Gal-5-3

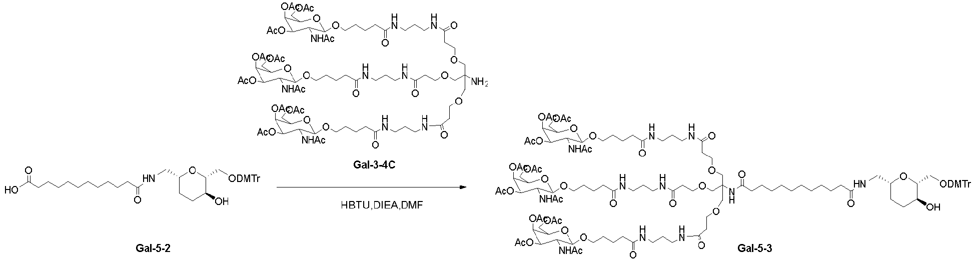

Dissolved Gal-5-2 (150 mg, 0.22 mmol) in 5 mL anhydrous DMF, and added HBTU (101 mg, 0.27 mmol), DIEA (0.16 mL), 3A molecular sieve (1 g), and Gal-3-4C (441 mg, 0.22 mmol) to this solution. Then stirred the above mixture at room temperature for 16 hours, and purified the reaction solution by reverse column C18, MeCN/ H₂O=60%, and obtain the product Gal-5-3 (380 mg).

¹H NMR (400 MHz, DMSO) *δ* 7.91 - 7.67 (m, 10H), 7.42 (d, 2H), 7.34 - 7.24 (m, 6H), 7.20 (d, 1H), 6.99 (s, 1H), 6.87 (d, 4H), 5.21 (d, 3H), 4.97 (dd, 3H), 4.60 (s, 1H), 4.48 (d, 3H), 4.09 - 3.95 (m, 10H), 3.93 - 3.82 (m, 3H), 3.77 - 3.66 (m, 9H), 3.61 - 3.48 (m, 12H), 3.45 - 3.20 (m, 16H), 3.09 - 2.95 (m, 15H), 2.27 (t, 6H), 2.10 (s, 9H), 2.05 (dd, 10H), 1.99 (s, 9H), 1.89 (s, 9H), 1.76 (d, 9H), 1.63 - 1.38 (m, 24H), 1.16 (s, 14H).

### Preparation of compound Gal-5-4

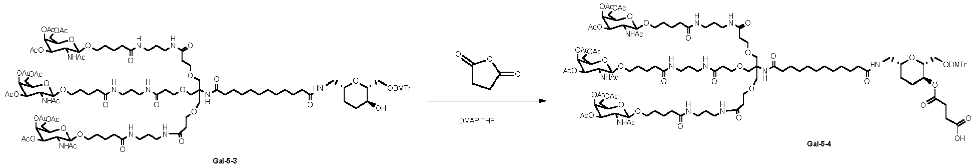

Dissolved Gal-5-3 (370 mg, 0.15 mmol), succinic anhydride (75 mg, 0.76 mmol), 3A molecular sieve (0.5 g), and DMAP (4-Dimethylaminopyridine) (46 mg, 0.38 mmol) in 5mL of THF (Tetrahydrofuran), then stirred overnight at 40 °C. Purified reaction solution through reverse column C18, MeCN/ H₂O=40%, and obtained the product Gal-5-4 (220 mg).

### 9) Synthesis of amino galactose compound GENO-Gal-5 connected to a solid-phase carrier

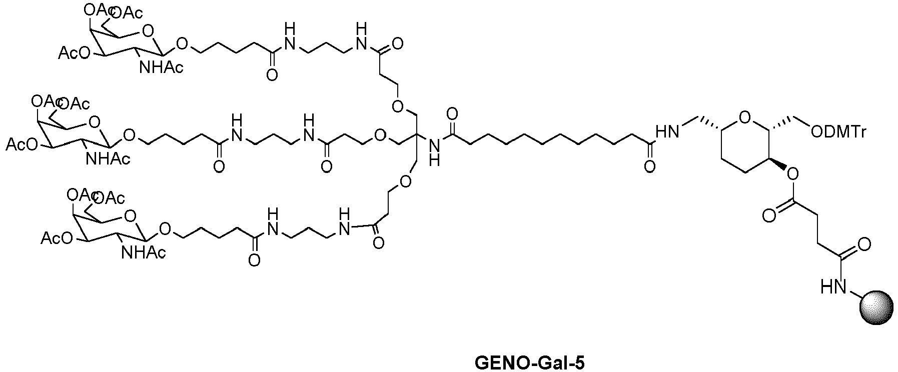

Suspended Gal-5-4 (220 mg, 0.086 mmol) in 4 ml CH₃CN and 2 mL DMF, added DIEA (0.035 mL, 0.216 mmol) and HBTU (49.07 mg, 0.129 mmol) dropwise, stirred at room temperature for 5 minutes, added ammonia methyl resin (99.51 mg, 100-200 mesh, amino load 250 umol/g) to the reaction solution, and conduct table concentrator reaction at 25°C at a speed of 220 rpm. After 16 hours of reaction, filtered and rinsed the filter cake with DCM three times, 30 ml each time, rinsed with acetonitrile three times, 30 ml each time, and rinsed with 30 ml n-hexane three times. Use a vacuum oil pump to dry the filter cake for 2 hours, then added a mixed reagent (CapB1, 4-dimethylaminopyridine, N-methylimidazole, and acetonitrile, 11.2 mL/12.4 mg/0.50 mL/4.32 mL) for cap reaction. Place the mixture on a table concentrator under 25 °C at a rotational speed of 220 rpm for 16 hours. Filtered the reaction solution, rinsed the filter cake with acetonitrile three times, 30 ml each time, filtered until dry, and dry overnight under vacuum oil pump pressure to obtain the target product, 160mg of GNEO-Gal-5 compound.

### 10)Preparation of compound Int-6-1

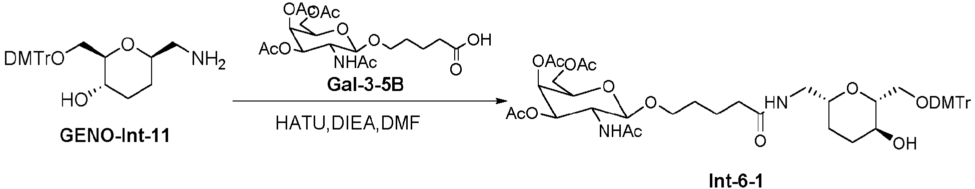

Dissolved Gal-3-5B (1.45 g, 3.24 mmol) in anhydrous DMF (10 mL) solvent, added HATU (1.64 g, 4.32 mmol), 3A molecular sieve (1 g), and DIEA (1.07 mL, 6.47 mmol). Stirred the reaction solution at room temperature for 30 minutes, then GENO-int-11 (1 g, 2.16 mmol) dissolved in DMF (10 mL) was added to the above reaction solution. Stirred the reaction solution overnight at room temperature under argon protection. Filtered the reaction solution and purified the product through reverse column C18, MeCN/H₂O=60%, and obtained the product Int-6-1 (1.8 g).

¹H NMR: (400 MHz, DMSO-*d₆*) *δ* 7.80 (d, *J* = 9.2 Hz, 1H), 7.71-7.73 (m, 1H), 7.39-7.41 (m, 2H), 7.24-7.30 (m, 6H), 7.18-7.21 (m, 2H), 6.87 (d, *J* = 8.8 Hz, 4H), 5.21 (d, *J* = 3.6 Hz, 1H), 4.95-4.98 (m, 1H), 4.59 (d, *J* = 6.4 Hz, 1H), 4.47 (d, *J* = 8.4 Hz, 1H), 4.00-4.05 (m, 2H), 3.83-3.90 (m, 1H), 3.64-3.73 (m, 7H), 3.23-3.38 (m, 8H), 2.97-3.14 (m, 3H), 2.04-2.14 (m, 5H), 1.98 (s, 3H), 1.89 (s, 3H), 1.77 (s, 3H), 1.64-4.66 (m, 1H), 1.42-1.50 (m, 4H).

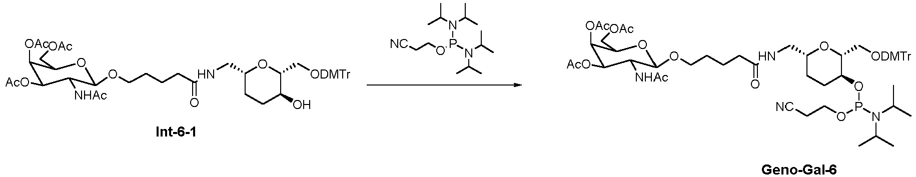

Dissolved Int-6-1 (2.1 g, 2.35 mmol) in DCM (30 mL), added Tetrazole (33 mg, 0.47 mmol), NMI (77.2 mg, 0.94 mmol), and 2 g molecular sieve. Displaced the reaction solution with argon gas three times, stirred at room temperature for 20 minutes, then dissolved the phosphorus reagent (920.81 mg, 3.06 mmol) in a small amount of dichloromethane and added it to the reaction solution. Stirred at room temperature for 1 hour. Washed the reaction solution twice with saturated NaHCO₃ aqueous solution, once with water, and once with salt water. Concentrate at room temperature and purified the product with a reverse column C18 MeCN/H₂O=65%, and obtained GENO Gal-6 (1.5 g).

¹H NMR: (400 MHz, CD₃CN) *δ* 7.48-7.50 (m, 2H), 7.20-7.36 (m, 7H), 6.83-6.87 (m, 4H), 6.45-6.51 (m, 1H), 5.28 (d, *J* = 3.2 Hz, 1H), 4.98-5.02 (m, 1H), 4.49 (d, *J* = 8.4 Hz, 1H), 3.90-4.12 (m, 4H), 3.24-3.76 (m, 20H), 3.02-3.11 (m, 1H), 2.49-2.59 (m, 1H), 2.36-2.39 (m, 1H), 2.08-2.25 (m, 9H), 1.97 (s, 3H), 1.91 (s, 3H), 1.83 (s, 3H), 1.71-1.74 (m, 1H), 1.46-1.63 (m, 5H), 0.85-1.39 (m, 20H).

### Example 2: Synthesis of compound GENO-Gal-7

### Synthetic route

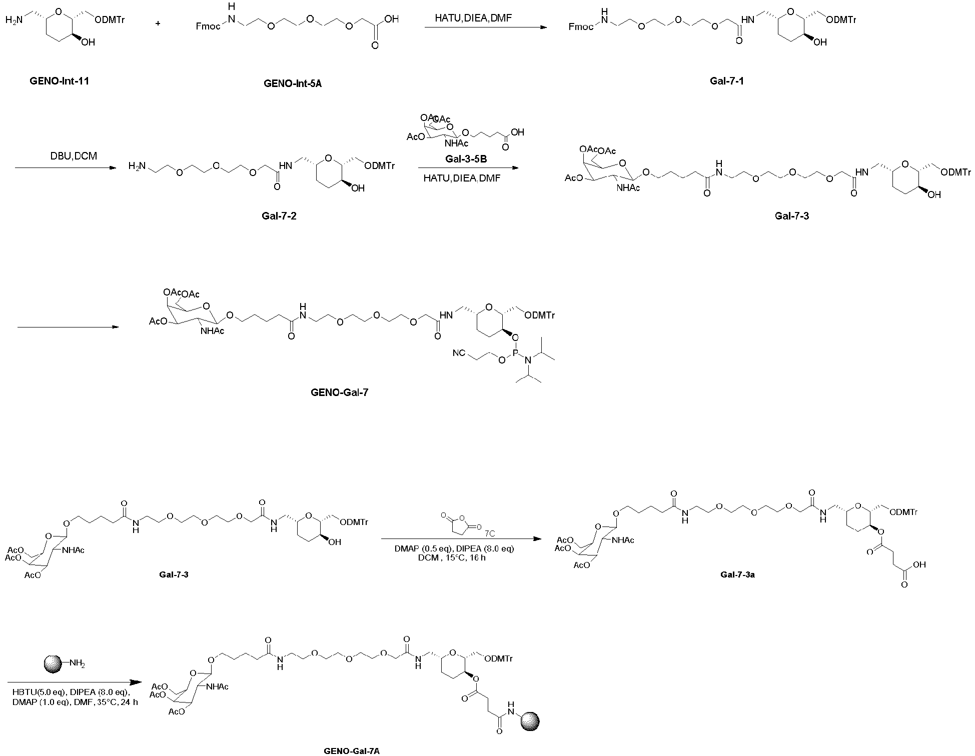

### Preparation of compound Gal-7-1

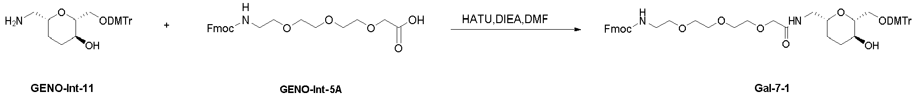

Dissolved **GENO-Int-SA** (5.91 g, 15.53 mmol) in anhydrous DMF (40 mL) solvent, added HATU (5.91 g, 15.53 mmol), 3A molecular sieve (3 g), and DIEA (6.42 mL, 38.83 mmol). Stirred the reaction solution at room temperature for 30 minutes, then dissolved **GENO-int-11** (3.6 g, 7.77 mmol) in DMF (10 mL) and added it to the above reaction solution. Stirred the reaction solution overnight at room temperature under argon protection. Filtered the reaction solution and purified the product through reverse column C18, MeCN/ H₂O=60%, and obtained the product **Gal -7-1**(4.7 g).

### Preparation of compound Gal-7-2

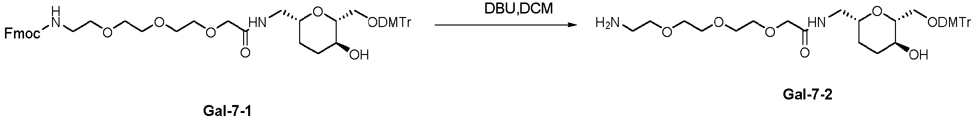

Dissolved Gal-7-1 (800 mg, 0.91 mmol) in 10 mL of dichloromethane, added DBU (0.27 mL, 1.83 mmol) to the above solution, stirred at room temperature for 1 hour, monitored by TLC, and the raw material was completely reacted. Quenched the reaction with 5 ml of saturated Na₂CO₃ solution, extract with 30 mL of dichloromethane, concentrated the organic phase, and purified with a reverse column C18 MeCN/H₂O=35% to obtain 330 mg of product. (The product was subjected to CH₃CN/H₂₀=20/1 silica gel thin-layer chromatography).

### Preparation of compound Gal-7-3

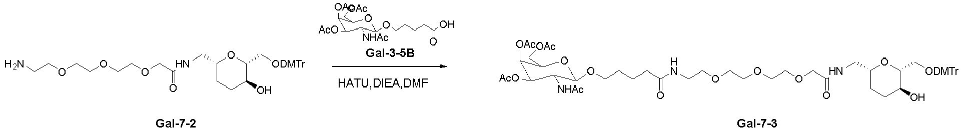

Dissolved **Gal-3-5B**(318.72 mg, 0.712 mmol) in anhydrous DMF (4 mL) solvent, added HATU (361.14 mg, 0.95 mmol), 3A molecular sieve (1 g), and DIEA (0.24 mL, 1.43 mmol). Stirred the reaction solution at room temperature for 30 minutes, then dissolved **Gal-7-2** (310 mg, 0.48 mmol) in DMF (3 mL) and added it to the above reaction solution. Stirred the reaction solution overnight at room temperature under argon protection. Filtered the reaction solution and purified the product through reverse column C18, MeCN/ H₂O=50%, and obtained the product **Gal-7-3**(370 mg).

### Preparation of compound GENO-Gal-7

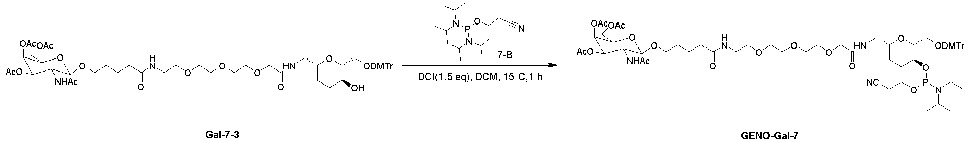

Dissolved **Gal-7-3**(3.0 g, 2.77 mmol) in DCM (30 mL), added **7-B** (1.34 g, 4.44 mmol), DCI(491 mg, 4.16 mmol), and 2 g molecular sieve. Displaced the reaction solution with argon gas three times, stirred at room temperature for 20 minutes, and stirred at room temperature for 1 hour. Washed the reaction solution twice with saturated NaHCO₃ aqueous solution, once with water, and once with salt water. Concentrated at room temperature and purified the product with a reverse column C18 MeCN/ H₂O=65%, and obtained **GENO -Gal-6**(1.5 g).
**¹H NMR:** *δ* 7.46-7.44 (d, *J* = 7.7 Hz 2H), 7.32-7.25 (m, 6H), 7.18-7.16 (m, 2H), 6.84-6.80 (m, 4H), 6.63-6.59(m, 2H), 5.25-5.24 (d, *J* = 3.2Hz 1H), 5.00-4.96 (dd, *J* = 11.2Hz, 3.3Hz ¹H ), 4.51-4.49 (d, *J* = 8.5 Hz 1H), 4.08-4.01 (m, 3H), 3.99-3.88 (m, 4H), 3.78-3.72 (m, 7H), 3.48-3.32 (m, 24H), 3.8-3.01 (m, 1H), 2.78-2.76 (m, 1H), 2.56-2.53 (m, 1H), 2.36-2.33 (m, 1H), 2.21-2.18 (m, 1H), 2.08-2.06 (m, 2H), 1.94(m, 4H), 1.91-1.87 (m, 4H), 1.80 (s, 3H), 1.70-1.67 (d, *J* = 13.1Hz 1H), 1.59-1.45 (m, 5H), 1.39-1.29 (m, 1H), 1.25-1.22 (m, 1H), 1.18-1.10 (m, 2H), 1.06-0.99 (m, 10H), 0.83-0.82 (d, *J* = 6.7Hz ,2H);
**³¹PNMR:** ET51671-213-P1O1 (162 MHz, CD₃CN);
*δ* 146.92, 146.03.

### 5) Preparation of compound Gal-7-3a

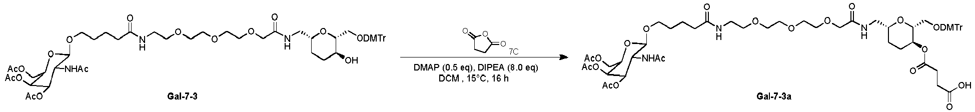

Dissolved Gal-7-3 (3.0 g, 2.77 mmol), succinic anhydride (2.22 g, 22.1 mmol), 3A molecular sieve (0.5 g), and triethylamine (2 mL, 3.8 mmol) in DCM (6 mL). Stirred reaction solution overnight at room temperature, filtered, washed with 5% NaCl solution, and concentrated at room temperature. Purified the product with a reverse column C18 MeCN/H₂O=40% to obtain the product Gal-7-3a (1.4 g).

### 6) Preparation of compound Gal-7-3a

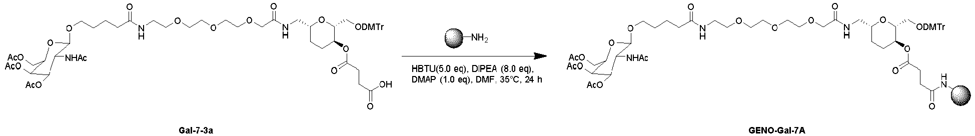

Suspended **Gal-7-3a**(0.7g, 0.6 mmol) in 8 mL acetonitrile and 4 mL N, N-dimethylformamide, added DIEA (0.83 mL, 4.74 mmol) and HBTU (1.12 g, 2.96 mmol) dropwise, stirred at room temperature for 5 minutes, added aminomethyl resin (409.43 mg, 100-200 mesh, amino load 250 umol/g) to the reaction solution, and conduct table concentrator reaction at 25°C at a speed of 220 rpm. After 16 hours of reaction, filtered and rinsed the filter cake with DCM three times, 30 ml each time, rinsed with acetonitrile three times, 30 ml each time, and rinsed with 30 ml n-hexane three times. Dried with vacuum oil pump for 2 hours, then added raw materials (CapB1, 4-dimethylaminopyridine, N-methylimidazole, and acetonitrile) according to the feeding ratio shown in Table 2 for capping reaction. Placed the mixture on a table concentrator under 25 °C at a rotational speed of 220 rpm for 16 hours of reaction. Filtered the reaction solution, rinsed the filter cake with acetonitrile three times, 30 ml each time, filtered until dry, and dried overnight under vacuum oil pump pressure to obtain 4.9 g **GNEO-Gal-7A** compound.

### Example 3: Synthesis of oligonucleotides conjugated with aminogalactose molecular clusters

The synthesis of siRNA is basically the same as the usual solid-phase synthesis method for phosphoramides, and the difference is that when the SS strand of siRNA is synthesized, the synthesized CPG carrier with amino galactose clusters is used instead of the usual Universal-CPG carrier. The synthesis process of siRNA (specific sequence shown in Table 1) was briefly described as follows: Dr. Oligo 48 synthesizer (Biolytic), starting from the Universal CPG carrier or the CPG carrier connected to the aminogalactose cluster, nucleoside phosphoramide monomers were sequentially connected according to the synthesis procedure. Raw materials for nucleoside phosphoramide monomers such as 2'- F RNA and 2'-O-methyl RNA were purchased from Wuhu Huaren and Shanghai Zhaowei. 5-ethylthio-1H-tetrazole (ETT) was used as an activator (0.6M acetonitrile solution), and 0.22M of PADS was dissolved in a mixture, with volume ratio of 1:1, of acetonitrile and trimethylpyridine (Shanghai Lingjiang). The resulting solution was used as sulfurization reagent, and iodopyridine/aqueous solution (Shanghai Lingjiang) was used as an oxidant.

After solid-phase synthesis, oligoribonucleotides were cleaved from the solid support and soaked in 28% ammonia solution at 50°C for 16 hours. After centrifugal treatment, the supernatant was transferred to another centrifuge tube, concentrated and evaporated to dryness, and purified with C18 reverse chromatography, with a mobile phase of 0.1M TEAA and acetonitrile. Target oligonucleotide, after collection, was repeatedly freeze-dried and identified as the target product by LC-MS, and then quantified by UV (260nm).

The obtained single stranded oligonucleotides were paired with complementary pairs according to equimolar ratios, annealed with AS strands, and finally the double stranded siRNA was dissolved in 1X PBS and adjusted to the required concentration for the experiment.

**Table 1 Oligonucleotide sequences (delivered sequences) in the present embodiment of the application used to inhibit ANGPTL3 expression**

| **RNAi agent number** | **Antisense strand** | **SEQ ID before modification** | **Sense strand** | **SEQ ID before modification** |
|---|---|---|---|---|
| Geno-2-102M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUm[L96] | 2 |
| Geno-2-104M | UmsAfsUm CmGmAf CmGmUm GmUmCm CmAfGm CfUmAm GmsUmsUm | 3 | AmsAmsCm UmAmGm CfUmGf GfAfCm AmCmGm UmCmGm AmUmAm[L96] | 4 |
| Geno-2-122M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUm[Gal-3] | 2 |
| Geno-2-123M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUm[Gal-5] | 2 |
| Geno-2-124M | AmsAfsAm AmAmGf AmCmUm GmAmUm CmAfAm AfUmAm UmGmUms UmsGm | 1 | [Gal-6]sAmsCmAm UmAmUm UfUmGf AfUfCm AmGmUm CmUmUm UmUmUm[Gal-6] | 2 |
| Geno-2-125M | AmsAfsAm AmAmGf AmCmUm GmAmUm CmAfAm AfUmAm UmGmUms UmsGm | 1 | [Gal-6]sAmsCmAm UmAmUm UfUmGf AfUfCm AmGmUm CmUmUm UmUmUms[Gal-6] | 2 |
| Geno-2-127M | AmsAfsAm AmAmGf AmCmUm GmAmUm CmAfAm AfUmAm UmGmUms UmsGm | 1 | [Gal-6]s[Gal-6]sAmCmAm UmAmUm UfUmGf AfUfCm AmGmUm CmUmUm UmUmUm[Gal-6][Gal-6] | 2 |
| Geno-2-128M | AmsAfsAm AmAmGf AmCmUm GmAmUm CmAfAm AfUmAm UmGmUms UmsGm | 1 | [Gal-6]s[Gal-6]sAmCmAm UmAmUm UfUmGf AfUfCm AmGmUm CmUmUm UmUmUms[Gal-6]s[Gal-6] | 2 |
| Geno-2-148M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUm[Gal-6][Gal-6][Gal-6] | 2 |
| Geno-2-149M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUm s[Gal-6]s[Gal-6]s[Gal -6] | 2 |
| Geno-2-150M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUms[Gal-3] | 2 |
| Geno-2-151M | AmsAfsAm AmAmGf AmCmUm GmAmUm CmAfAm AfUmAm UmGmUms UmsGm | 1 | AmsCmsAm UmAmUm UfUmGf AfUfCm AmGmUm CmUmUm UmUmUms[Gal-5] | 2 |

**Table 2 Oligonucleotide sequences (delivered sequences) in the present embodiment of the application for detecting the distribution of LPA siRNA in the liver**

| RNAi agent No. | Antisense strand 5'→3' | SEQ ID NO. before modification | Sense strand 5'→3' | SEQ ID NO. before modification |
|---|---|---|---|---|
| Geno-1-105M | AmsUfsAm AmCfUmCmUmGm UmCmCm AmUfUm AfCmCm AmUmGms GmsUm | 5 | [Gal-6] sCmsAmsUm GmGmUm AmAmUf GfGfAm CmAmGm AmGmUm UmAmUms[Gal-6] | 6 |
| Geno-1-106M | AmsUfsAm AmCfUmCmUmGm UmCmCm AmUfUm AfCmCm AmUmGms GmsUm | 5 | [Gal-6]s[Gal-6]sCmAmUm GmGmUm AmAmUf GfGfAm CmAmGm AmGmUm UmAmUm[Gal-6][Gal-6] | 6 |
| Geno-1-107M | AmsUfsAmAmCfUmC mUmGm UmCmCm AmUfUm AfCmCm AmUmGms GmsUm | 5 | CmsAmsUm GmGmUm AmAmUf GfGfAm CmAmGm AmGmUm UmAmUms[Gal-6]s[Gal-6]s[Gal -6] | 6 |
| Geno-1-108M | AmsUfsAm AmCfUmCmUmGm UmCmCm AmUfUm AfCmCm AmUmGms GmsUm | 5 | CmsAmsUm GmGmUm AmAmUf GfGfAm CmAmGm AmGmUm UmAmUms[Gal-5] | 6 |
| Geno-1-1004M | AmsUfsAm AfCmUf CmUfGm UfCmCf AmUfUm AfCmsCfs Gm | 7 | [ST23sST23sST23sC6XLT] sCfsGmsGf UmAfAmUfGmGfAmCfAmGf AmGfUmUfsAmsUf | 8 |

### L96 structural formula:

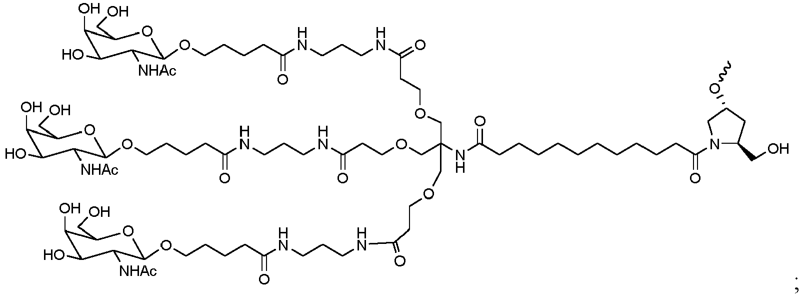

### Gal-3 structural formula:

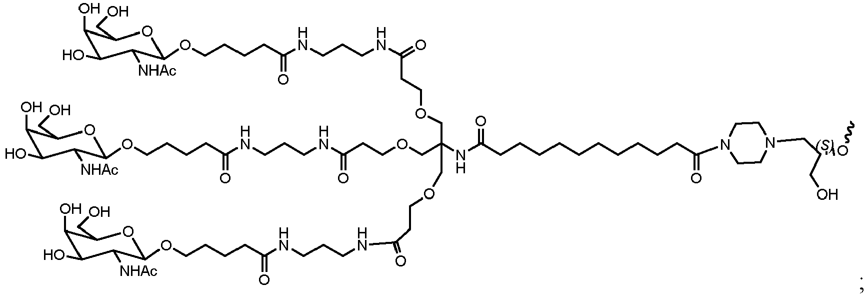

### Gal-5:

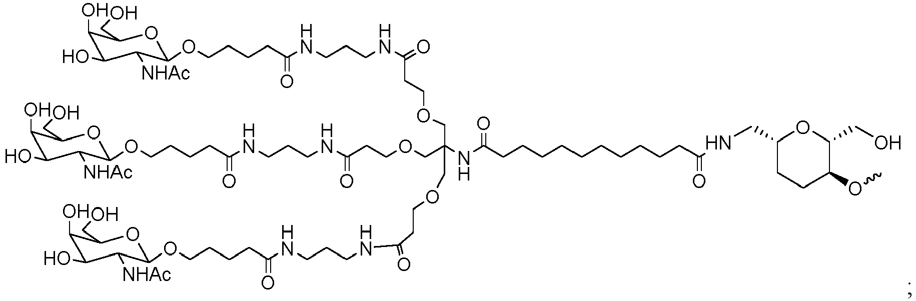

### Gal-6:

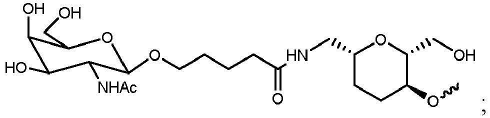

### [ST23sST23sST23sC6XLT] structural formula:

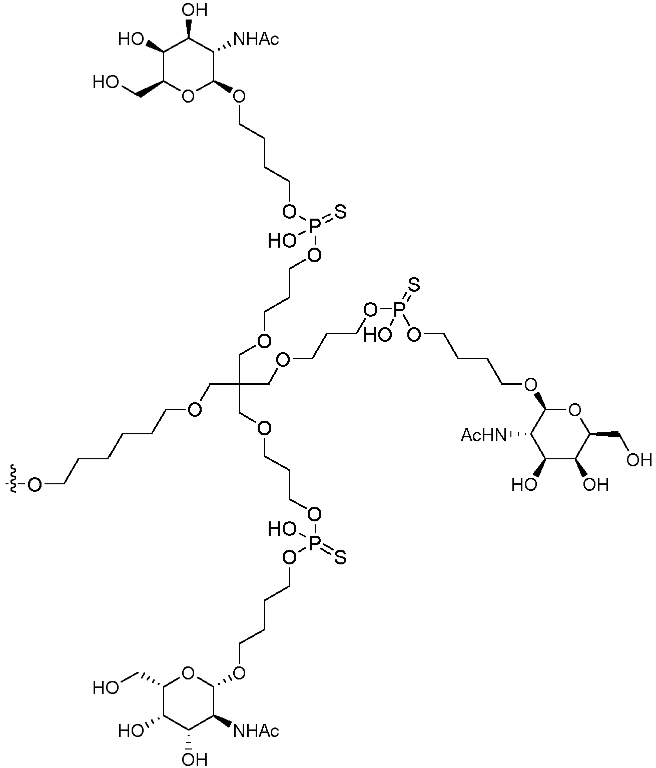

In the sequences in Table 1 and Table 2, symbols are used to represent the following modified nucleotides (esters), respectively:
A=Adenosine-3 '- phosphate ester
C=Cytidine 3 '- phosphate ester
G=guanosine-3 '- phosphate ester
U=Urine-3 '- phosphate ester
Am=2 '- O-methyladenosine-3' - phosphate ester
Ams=2 '- O-methyladenosine-3' - thiophosphate ester
Cm=2 '- O-methylcytidine-3' - phosphate ester
Cms=2 '- O-methylcytidine-3' - thiophosphate ester
Gm=2 '- O-methylguanosine-3' - phosphate ester
Gms=2 '- O-methylguanosine-3' - thiophosphate ester
Um=2 '- O-methyluridine 3' - phosphate ester
Ums=2 '- O-methylurine-3' - thiophosphate ester
Af=2 '- fluoroadenosine-3' - phosphate ester
Afs=2 '- fluoroadenosine-3' - thiophosphate ester
Cf=2 '- fluorocytidine-3' - phosphate ester
Cfs=2 '- fluorocytidine-3' - thiophosphate ester
Gf=2 '- fluoroguanosine-3' - phosphate ester
Gfs=2 '- fluoroguanosine-3' - thiophosphate ester
Uf=2 '- fluorouridine-3' - phosphate ester
Ufs=2 '- fluorouridine-3' - thiophosphate ester

Where: The lowercase letter m indicates that the adjacent nucleotide to the left of the letter m is a methoxy modified nucleotide; the lowercase letter f indicates that the adjacent nucleotide to the left of the letter f is a fluorinated nucleotide;
The lowercase letter s in the middle of uppercase letters indicates that the connection between two adjacent nucleotides to the left and right of the letter s is a thiophosphate group connection;
The lowercase letter s which is the first one at the 3 'end indicates that the nucleotide end adjacent to the left of the letter s is a thiophosphate group;

In oligonucleotides, nucleotide monomers are interconnected through 5 '-3' - phosphodiester bonds, including thiophosphate bonds and phosphodiester bonds.

### Example 4: ANGPTL3 RNAi agent inhibits Angptl3 mRNA expression in mice

Male C57 BL/6 mice were used to evaluate the in vivo activity of ANGPTL3 RNAi. After adaption to environmental facilities, the mice were evenly grouped based on their weight with 3 mice in each group. On the day of administration (day 0), the mice were subcutaneously injected with ANGPTL3 RNAi solution, while the control group mice were given PBS solution. All mice were subcutaneously injected once in the flank of back, with a dosage of 1mg/Kg and a volume of 5ml/Kg. 3 days before administration, and on day 5, 14, 21, and 28 after administration, blood was collected from all mice through the orbital venous plexus (after isoflurane anesthesia) for serum collection, and blood collection continued for some mice in the administration group on day 35 and 42. The animals were euthanized after blood collection.

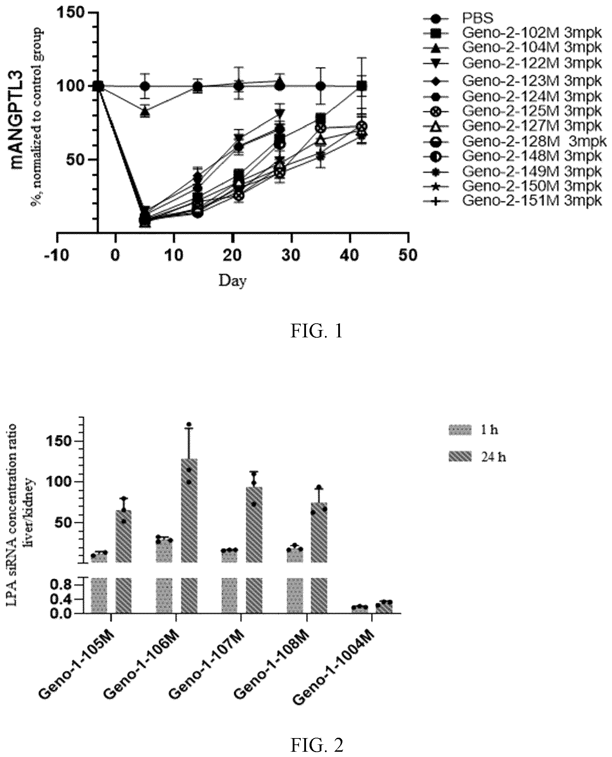

Evaluate the knockdown of mANGPTL3 expression by detecting the mANGPTL3 protein in mouse serum using ELISA method (mANGPTL3, R&D). For the purpose of normalization, the mANGPTL3 level of each animal at a given time point was divided by the animal's pre-treatment level (Day-3) to determine the expression ratio of "normalized to pre-treatment". The expression at a specific time point was normalized to the control group by dividing each animal's "normalized to pre-treatment" ratio by the average "normalized to pre-treatment" ratio of all mice in the control group. The expression at each time point can be normalized to the expression in the control group, and the specific detection results after normalization were shown in Figure 1.

ANGPTL3 RNAi agent (including the oligonucleotide sequences described in Table 1) was administered to C57BL/6 mice as described above. Each mouse received a single subcutaneous injection (SC) of 1mg/Kg ANGPTL3 RNAi solution, and mANGPTL3 protein in serum was monitored for 42 days. The knock-down level and response duration are shown in Table 3. Table 3 shows that on day 5 of administration, 11 ANGPTL3 RNAis showed knockdown higher than 80% at 1mg/kg, 6 of which showed knockdown higher than 90%; on day 14, 8ANGPTL3 RNAis showed knockdown higher than 70% at 1mg/kg, 5 of which showed knockdown higher than 80%; on day 21, 8 ANGPTL3 RNAis showed knockdown higher than 50% at 1mg/kg, 3 of which showed knockdown higher than 70%; on day 28, 5 ANGPTL3 RNAi maintained knockdown higher than 50% in mANGPTL3 protein; on day 35, 2 ANGPTL3 RNAis maintained knockdown higher than 45% in mANGPTL3 protein.

Angiopoietin-like 3 (ANGPTL3) is an angiopoietin protein encoded by the human angiopoietin-like 3 gene, mainly expressed in the liver and used to regulate lipid metabolism. An effective therapeutic agent targeting ANGPTL3 can be used to treat (including preventive treatment) metabolic diseases, such as hypertriglyceridemia. The experimental results in Table 3 indicated that the targeting ligand in this application can efficiently deliver oligonucleotide sequences to liver cells and inhibit the expression of ANGPTL3.

**Table 3. Relative mANGPTL3 levels in mouse serum after subcutaneous administration of 1mg/Kg ANGPTL3 RNAi**

| Treatment | Day3 | | Day 5 | | Day 14 | | Day 21 | | Day 28 | | Day 35 | | Day 42 | |
|---|---|---|---|---|---|---|---|---|---|---|---|---|---|---|
| | Average value | Standard deviation | Average value | Standard deviation | Average value | Standard deviation | Average value | Standard deviation | Average value | Standard deviation | Average value | Standard deviation | Average value | Standard deviation |
| PBS | 1.00 | 0.00 | 1.00 | 0.14 | 1.00 | 0.08 | 1.00 | 0.07 | 1.00 | 0.05 | 1.00 | 0.21 | 1.00 | 0.12 |
| Geno-2-102M | 1.00 | 0.00 | 0.10 | 0.03 | 0.25 | 0.08 | 0.40 | 0.07 | 0.64 | 0.10 | 0.78 | 0.06 | 1.00 | 0.33 |
| Geno-2-104M | 1.00 | 0.00 | 0.83 | 0.07 | 1.00 | 0.03 | 1.02 | 019 | 1.03 | 0.08 | \ | \ | \ | \ |
| Geno-2-122M | 1.00 | 0.00 | 0.15 | 0.05 | 0.35 | 0.16 | 0.64 | 0.11 | 0.81 | 0.12 | \ | \ | \ | \ |
| Geno-2-123M | 1.00 | 0.00 | 0.13 | 0.02 | 0.39 | 0.10 | 0.59 | 0.08 | 0.72 | 0.07 | \ | \ | \ | \ |
| Geno-2-124M | 1.00 | 0.00 | 0.13 | 0.02 | 0.31 | 0.01 | 0.59 | 0.10 | 0.71 | 0.04 | \ | \ | \ | \ |
| Geno-2-125M | 1.00 | 0.00 | 0.09 | 0.02 | 0.22 | 0.06 | 0.26 | 0.08 | 0.41 | 0.12 | 0.72 | 0.13 | 0.73 | 0.21 |
| Geno-2-127M | 1.00 | 0.00 | 0.08 | 0.03 | 0.22 | 0.06 | 0.35 | 0.12 | 0.48 | 0.08 | 0.64 | 0.03 | 0.70 | 0.15 |
| Geno-2-128M | 1.00 | 0.00 | 0.10 | 0.00 | 0.14 | 0.04 | 0.32 | 0.01 | *0.44* | 0.11 | \ | \ | \ | \ |
| Geno-2-148M | 1.00 | 0.00 | 0.08 | 0.01 | 0.17 | 0.03 | 0.35 | 0.08 | 0.61 | 0.23 | | \ | \ | \ |
| Geno-2-149M | 1.00 | 0.00 | 0.08 | 0.01 | 0.16 | 0.03 | 0.31 | 0.10 | 0.41 | 0.06 | 0.52 | 0.13 | 0.66 | 0.04 |
| Geno-2-150M | 1.00 | 0.00 | 0.10 | 0.03 | 0.16 | 0.07 | 0.29 | 0.07 | 0.50 | 0.10 | \ | \ | \ | \ |
| Geno-2-151M | 1.00 | 0.00 | 0.09 | 0.01 | 0.14 | 0.01 | 0.26 | 0.02 | 0.45 | 0.02 | 0.55 | 0.04 | 0.74 | 0.11 |

### Example 5: Distribution ratio of LPA RNAi agents in wild mouse liver and kidney after subcutaneous administration

30 C57BL6/J mice were divided into 10 groups, and 5 designated groups of LPA RNAi agents (including oligonucleotide sequences in Table 2) were subcutaneously injected into each group of mice at a dose of 10mg/kg on the first day. Five groups of mice underwent tissue collection 1 hour after administration, while the other five groups underwent tissue collection 24 hours after administration. The collected tissue samples were frozen quickly and transferred to -80 °C for storage. After tissue homogenization, the concentration of corresponding LPA RNAi agents in liver tissue homogenization and kidney tissue homogenization was measured using a hybrid fluorescence probe enzyme-linked immunosorbent assay method. The liver kidney ratio was calculated by dividing the LPA RNAi concentration of liver tissue homogenization by that of kidney tissue homogenization. The results are shown in Table 4 and Figure 2.

Figure 2 shows the concentration ratio of compounds in the liver and kidney of wild mice after s.c. administration of 10mg/kg LPA RNAi . The results indicated that the concentration of Geno-1-105M-Geno-1-108M in liver ( target organ), was significantly higher than that in kidney (non-targeting organ), at different time points, and the drug concentration ratio in the liver to the kidney was much greater than 1. Meanwhile, at the two time points of testing, the concentration of Geno-1-1004M in the non-targeting organ kidney was higher than that in the liver, and the drug concentration ratio in the liver to the kidney was less than 1.

**Table 4: Concentration ratio of compounds in the liver and kidney of wild mice after administration of 10mg/kg LPA RNAi by SC**

| | 1-hour average liver to kidney ratio | SD% | 24-hour average liver to kidney ratio | SD% |
|---|---|---|---|---|
| Geno-1-105M | 12.1 | 21% | 65.9 | 30% |
| Geno-1-106M | 29.5 | 14% | 128.6 | 39% |
| Geno-1-107M | 16.5 | 1% | 94.2 | 8% |
| Geno-1-108M | 19.3 | 24% | 74.8 | 25% |
| Geno-1-1004M | 0.2 | 13% | 0.3 | 21% |

Lipoprotein (a) [Lp(a)] is a heterogeneous low-density lipoprotein (LDL) - like particle, and the LPA (Apo(a)) gene is mainly expressed in the liver. High Lp(a) level is an independent risk factor for cardiovascular disease, stroke and other related diseases (including atherosclerotic stenosis). The above embodiments demonstrate that the targeting ligand of the present application can efficiently deliver oligonucleotide sequence to liver cells. In Table 4, compared to the control, Geno-1-1004M allows the accumulation of more siRNAs in the kidneys, while other ligands mainly deliver siRNAss to the liver.

The above embodiments, which are preferred ones of the present invention only, are not intended to limit the present invention. The modifications, equivalent substitutions, and improvements made within the principles of the present invention shall be included within the scope of protection hereof.

## Claims

1. A cleavable compound, as shown in formula (Z-1), or pharmaceutically acceptable salt thereof 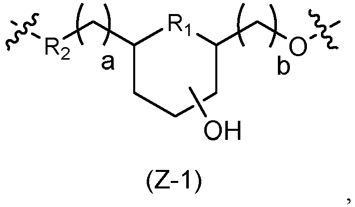
where R₁ is O, S, NR₃, or CR₃R₄, and R₃ and R₄ are independent hydrogen, halogen, substituted or unsubstituted aliphatic group, substituted or unsubstituted aryl group, substituted or unsubstituted heteroaryl group, substituted or unsubstituted heterocyclic group, or substituted or unsubstituted cycloalkyl group;
where R₂ is -O-, -S-, -NH-, -CH₂-, -C(O)-, -OC(O)-, -C(O)O-, -NHC(O)-, -C(O)NH-, -CH₂NH-, -CH₂O-, -NH-C(O)-CH₂-, -C(O)-CH₂-NH-, or -NH(CO)NH-, wherein the - CH₂- is optionally replaced with a substituent group selected from the group consisting of halogen, alkyl, alkoxy, and alkylamino; a and b are the same or different, and are the integers respectively selected from 0 to 20, preferably from 1 to 10, and further preferably from 1 to 5.

2. The cleavable compound or pharmaceutically acceptable salt thereof according to claim 1, wherein the compound has a structural formula of: 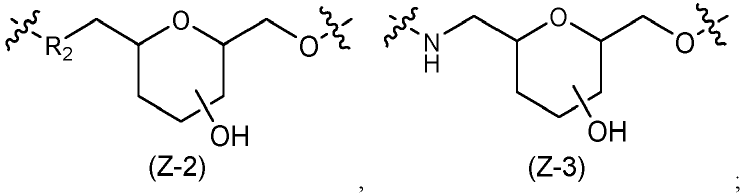 or

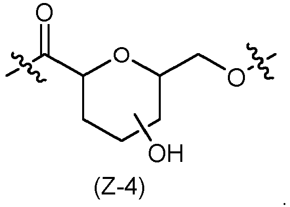

3. An oligonucleotide ligand compound or pharmaceutically acceptable salt thereof, comprising the cleavable compound or a pharmaceutically acceptable salt thereof according to claim 1 or 2, and the oligonucleotide ligand compound has a general formula of: 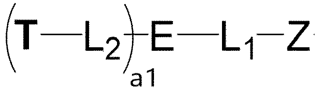 (I) or has a general formula of: 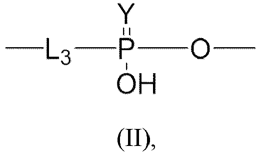 in the general formula (I):
Z is the cleavable compound for connection to nucleotide sequence X, L₁ is the second linker, E is the branch point group, the branch point group E is connected to a1 targeting combination(s), and a1 is an integer from 0 to 10, preferably from 1 to 5; the targeting combination comprises a tethered portion L₂ and a targeting portion T in a 1:1 ratio; or
in the general formula (II), Y is O, S, or N, and L₃ has a following structure: 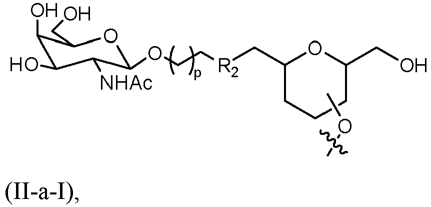 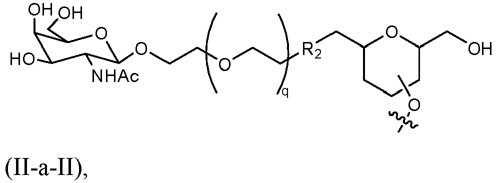 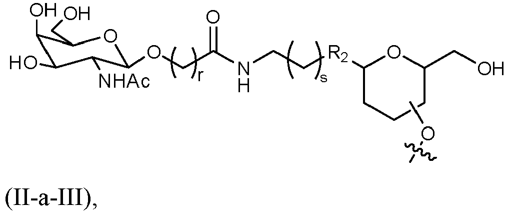 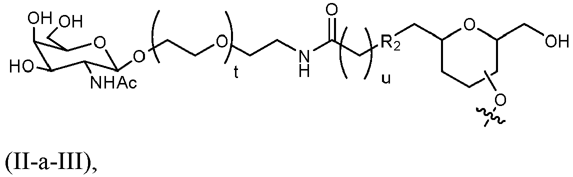 where R₂ is -O-, -S-, -NH-, -CH₂-, -C(O)-, -OC(O)-, -C(O)O-, -NHC(O)-, -C(O)NH-, -CH₂NH-, -CH₂O-, -NH-C(O)-CH₂-, -C(O)-CH₂-NH- or -NH(CO)NH-, and -CH₂- is optionally replaced with a substituent group of halogen or alkyl, wherein the alkyl is optionally further replaced with a substituent group selected from the group consisting of hydroxyl, amino, halogen, alkoxy, and alkylamino;
the p, q, r, s, t, and u are integers from 0 to 20, preferably from 1 to 10.

4. The oligonucleotide ligand compound or pharmaceutically acceptable salt thereof according to claim 3, wherein the general formula (II) is used for connecting with a nucleotide sequence.

5. The oligonucleotide ligand compound or pharmaceutically acceptable salt thereof according to claim 3, wherein the second linker portion L₁ in the general formula (I) has a following structure: 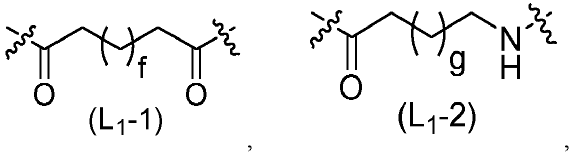 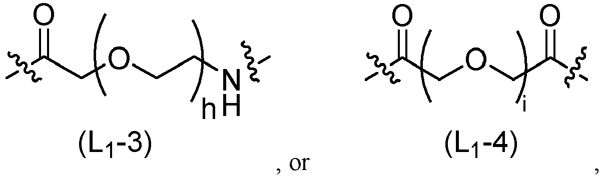 and the f, g, h, and i are integers from 1 to 20, preferably from 1 to 10, and further preferably from 1 to 5.

6. The oligonucleotide ligand compound or pharmaceutically acceptable salt thereof according to claim 3, wherein the second linker portion L₂ in the general formula (I) has a following structure: 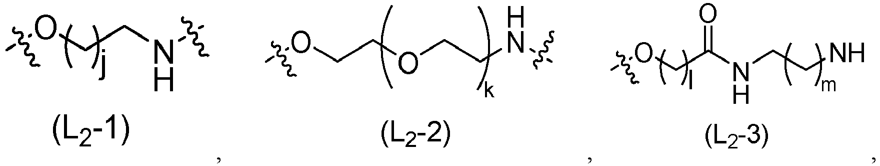 or 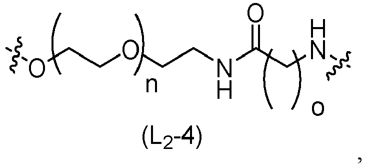 where j, k, l, m, n, and o are integers from 1 to 20, preferably from 1 to 10, and further preferably from 1 to 5.

7. The oligonucleotide ligand compound or pharmaceutically acceptable salt thereof according to claim 3, wherein the targeting portion T is selected from a tissue-specific targeting ligand, preferably liver specific targeting ligand; further preferably, the targeting portion T has a structure for enhancing the uptake of oligomers for liver cells.

8. The oligonucleotide ligand compound or pharmaceutically acceptable salt thereof according to claim 3, wherein the targeting portion T has the same or different structure as/from L₃.

9. The oligonucleotide ligand compound or pharmaceutically acceptable salt thereof according to claims 3-8, wherein the ligand compound has a following structure: 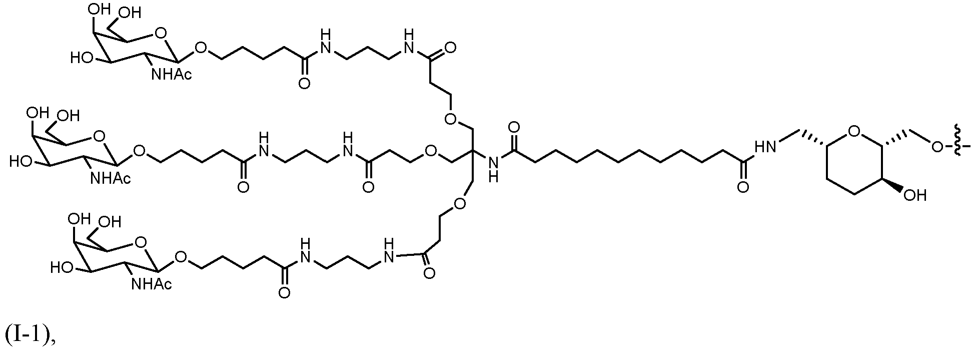 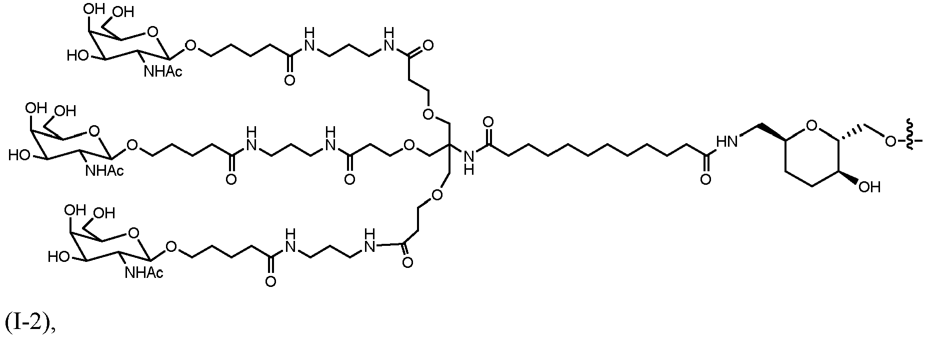 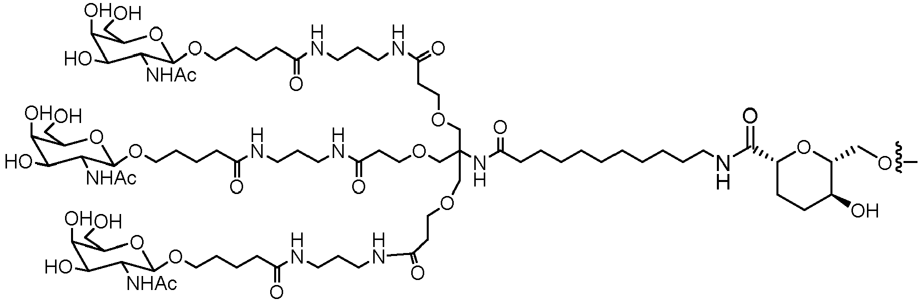 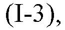 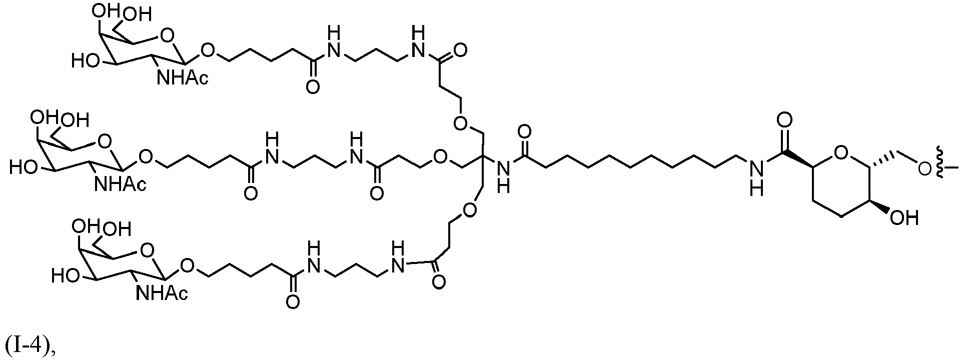 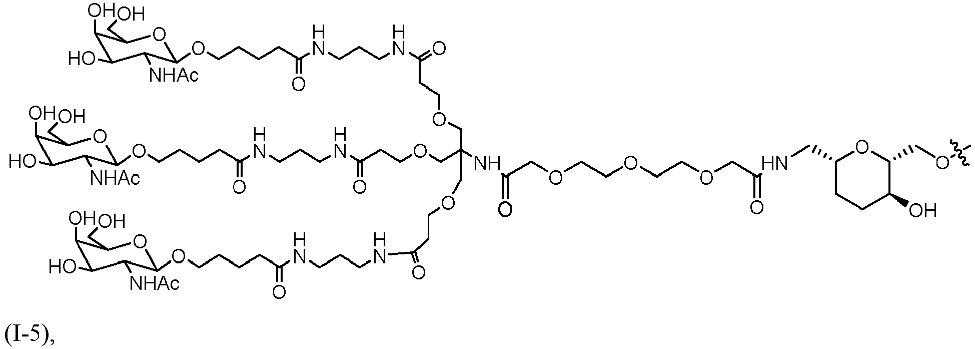 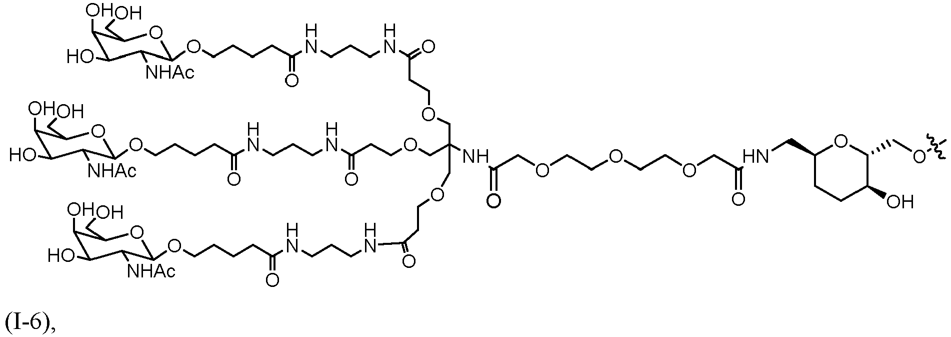 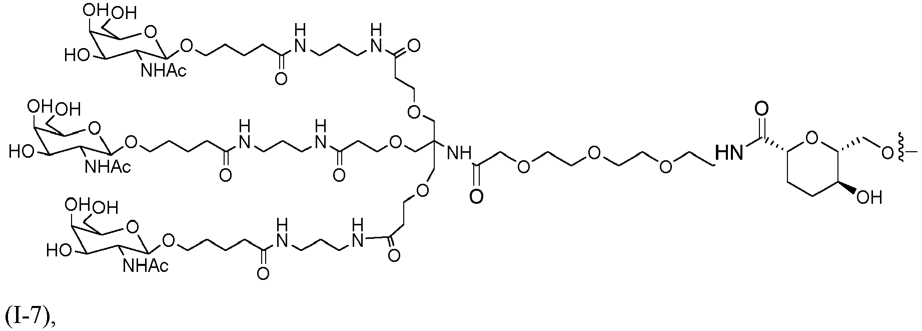 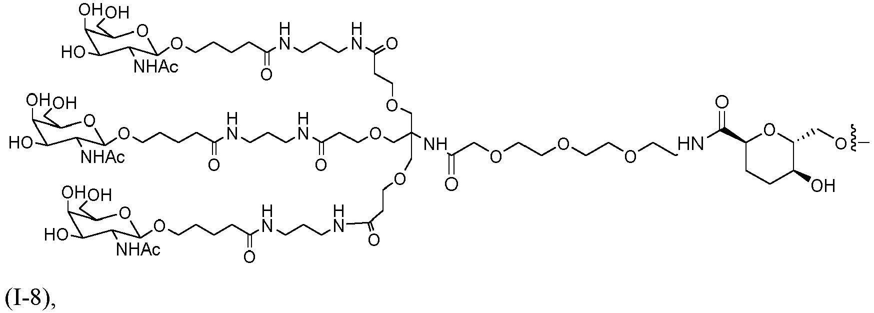 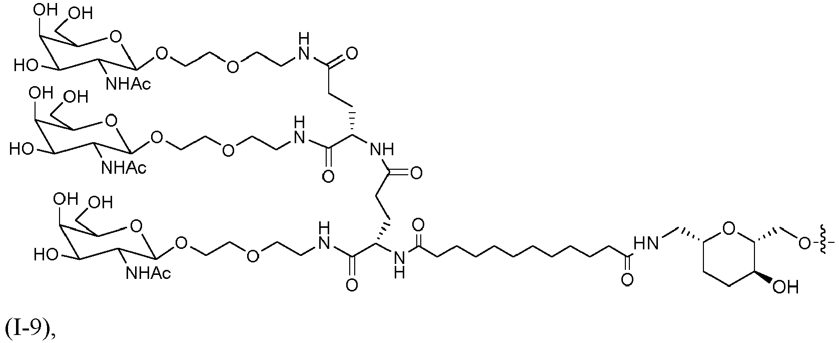 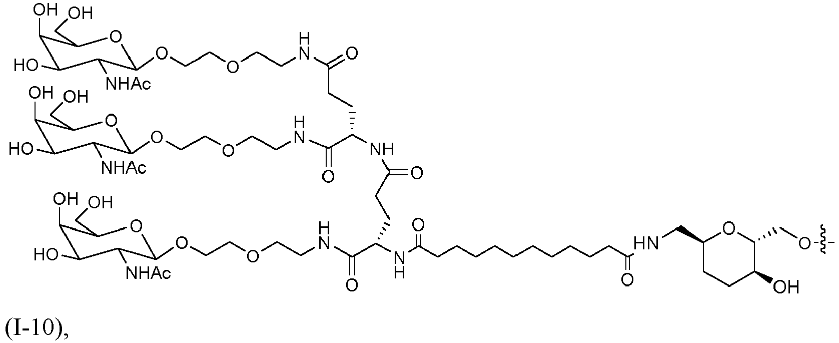 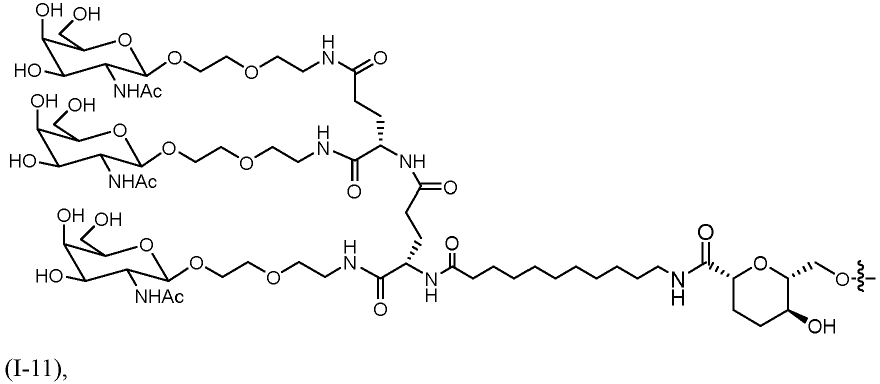 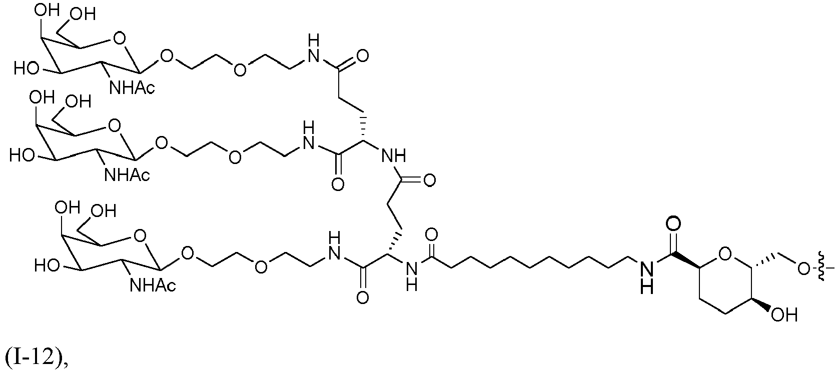 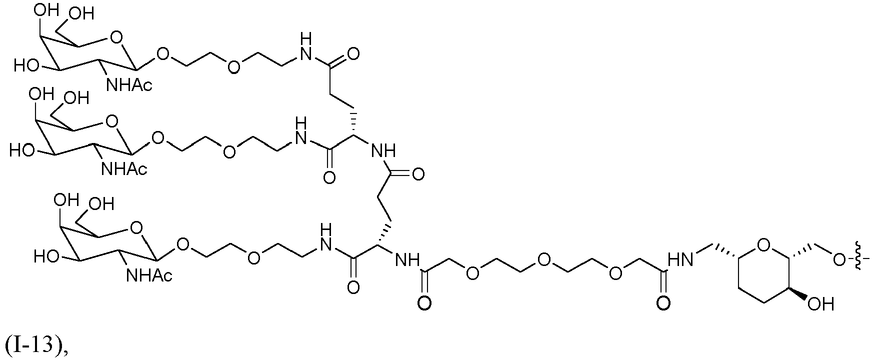 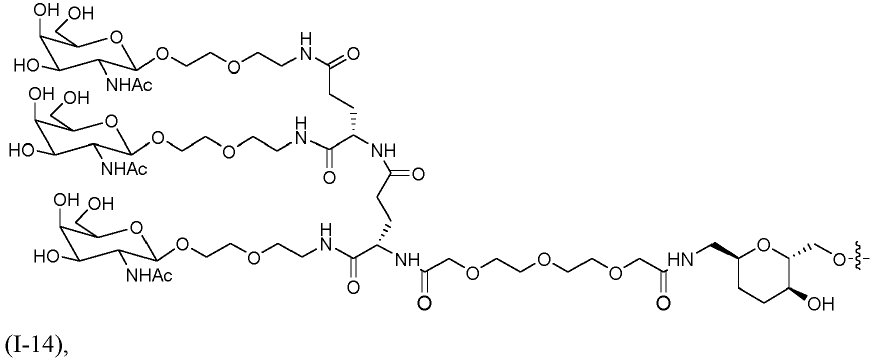 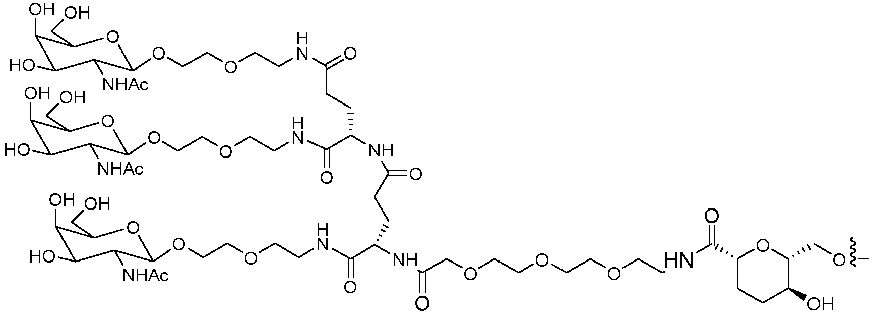 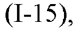 or

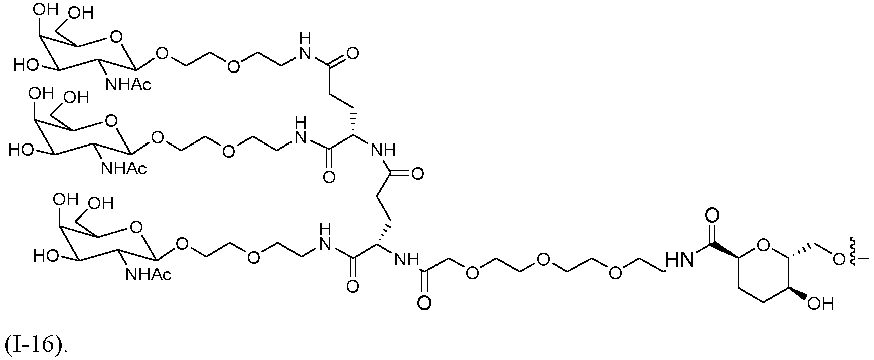

10. The oligonucleotide ligand compound or pharmaceutically acceptable salt thereof according to claims 3-8, wherein the ligand compound comprises a following structure: 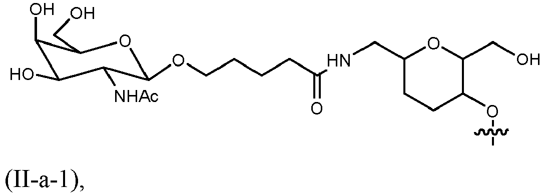 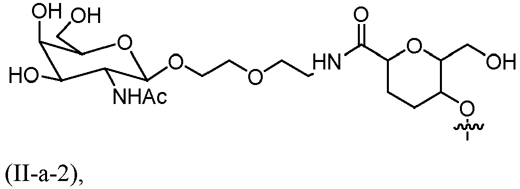 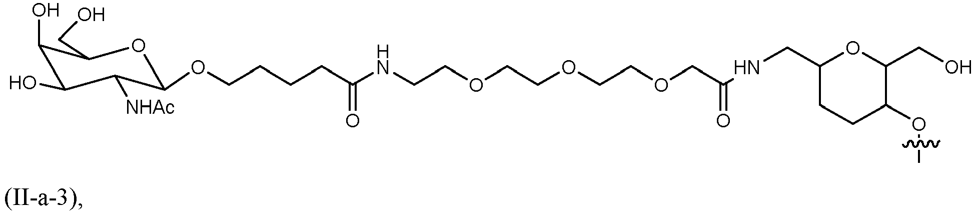 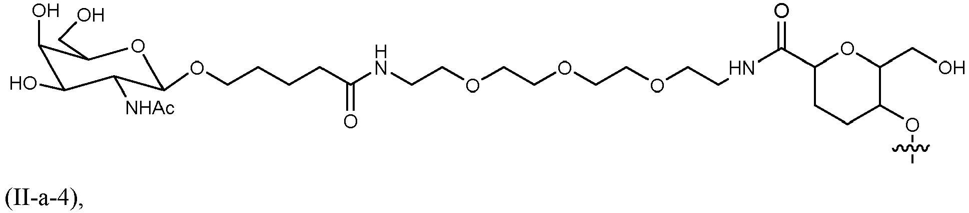 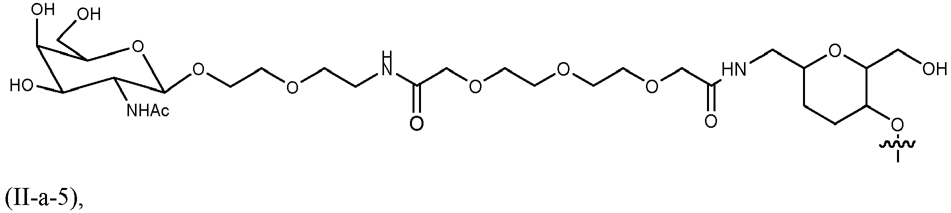 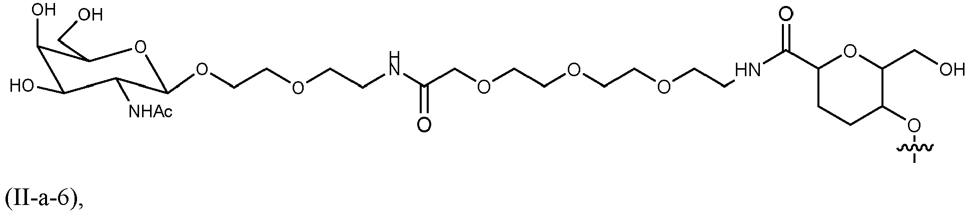 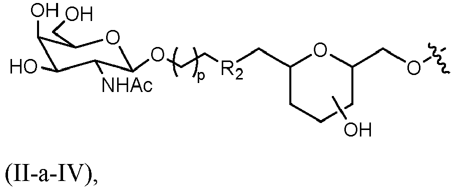 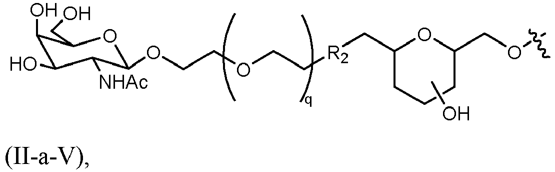 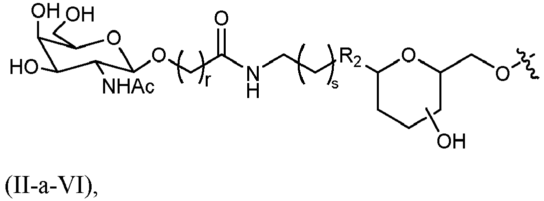 or

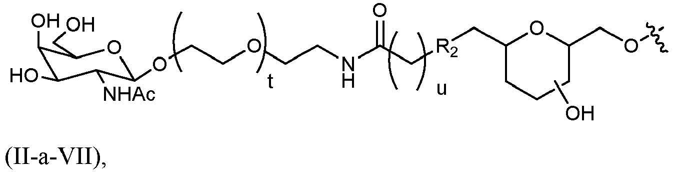

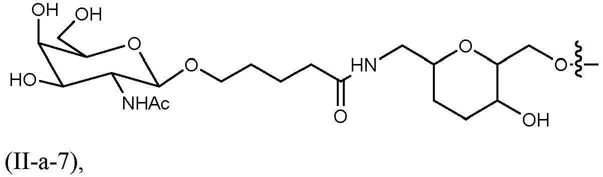

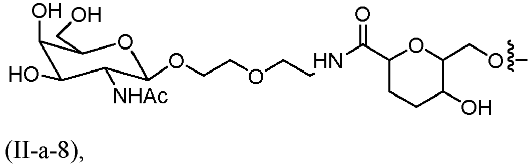

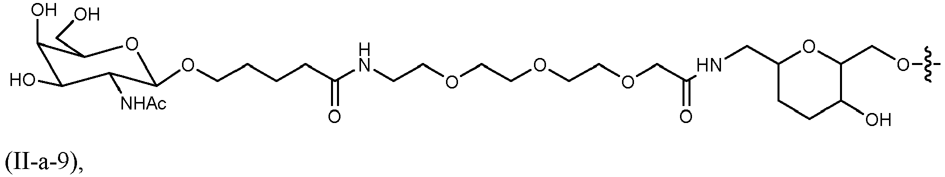

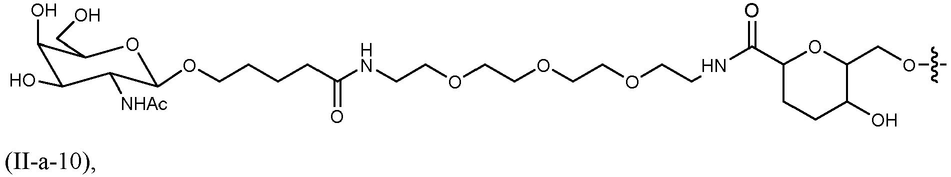

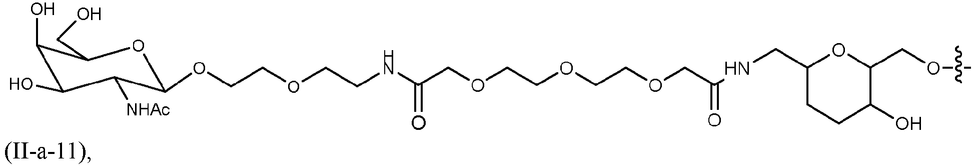

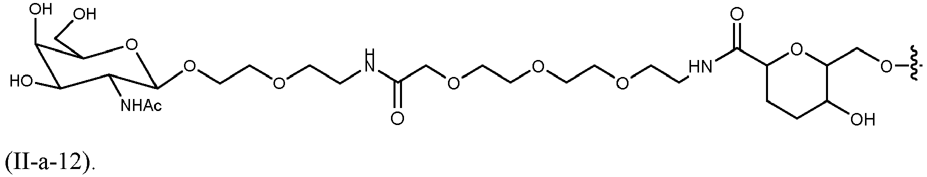

11. The oligonucleotide ligand compound or pharmaceutically acceptable salt thereof according to claims 3-8, wherein the ligand compound has the following structure after binding to nucleotide sequence X: 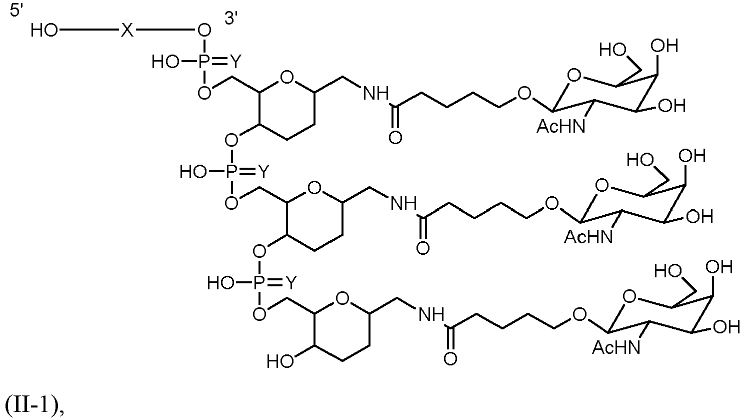 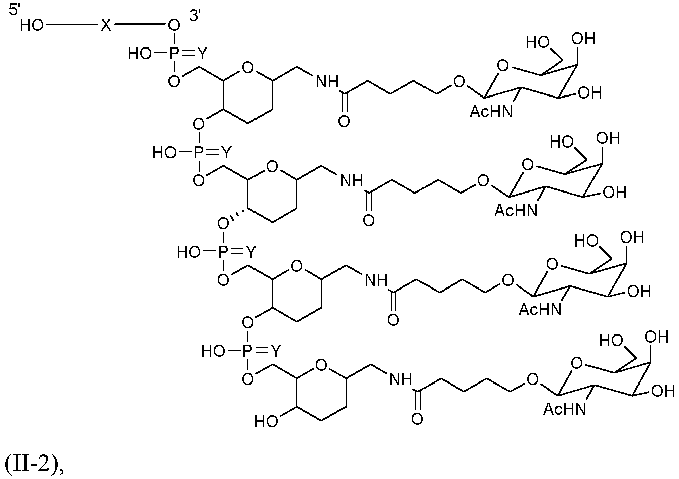 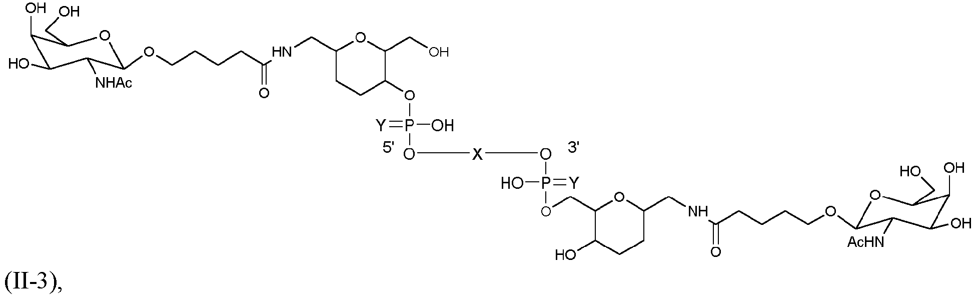 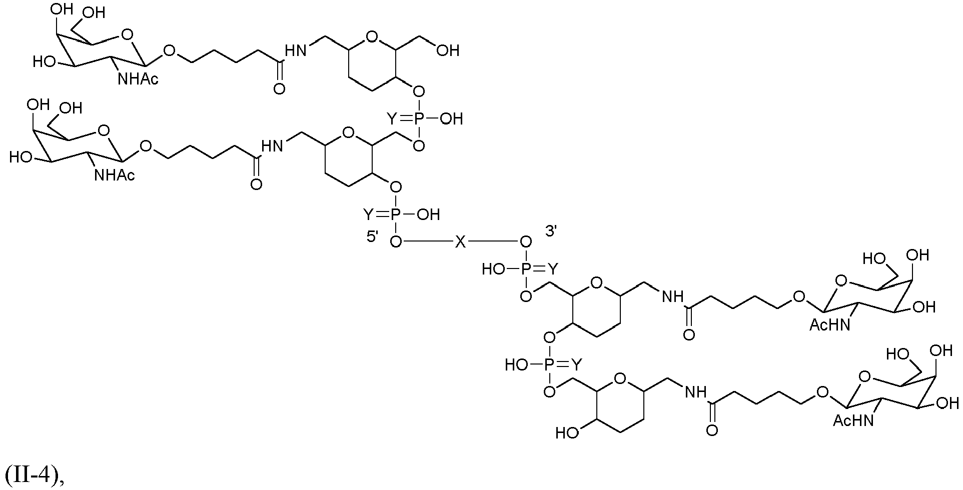 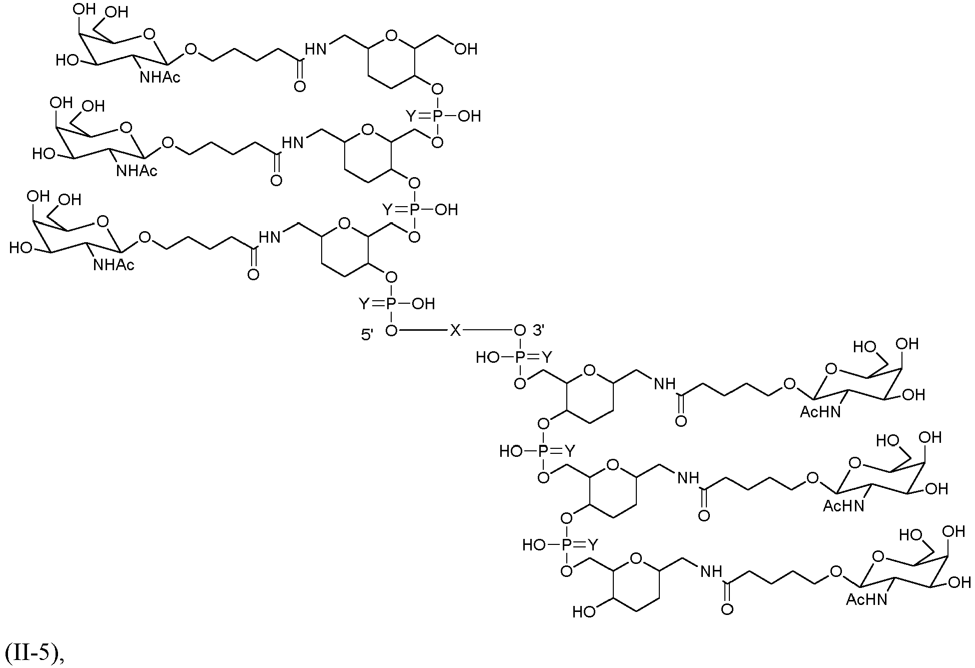 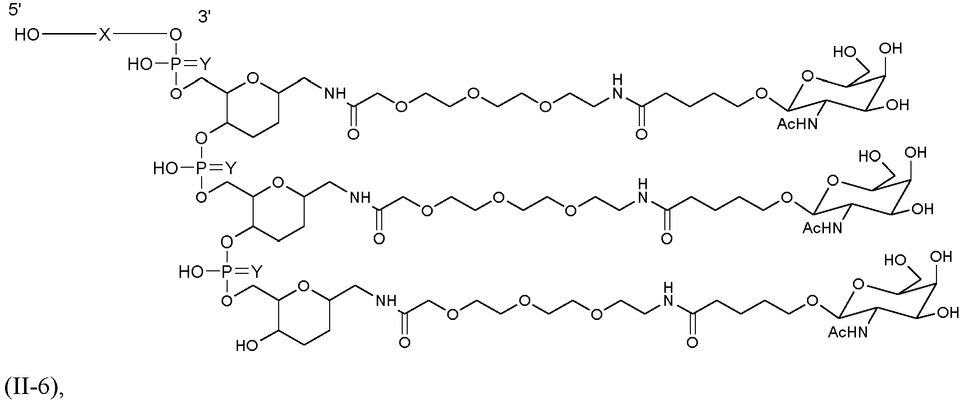 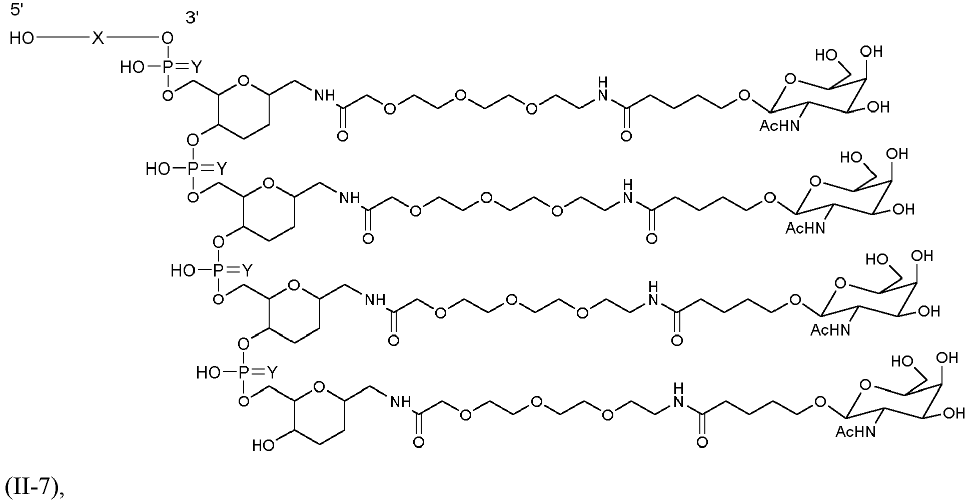 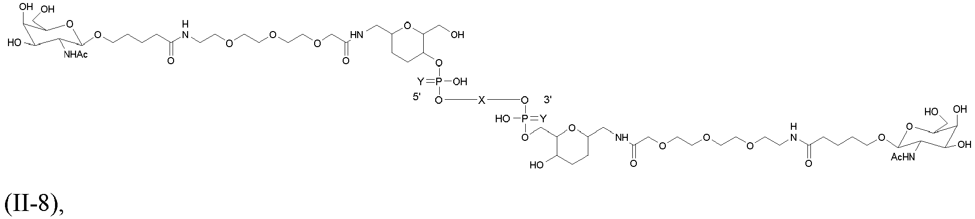 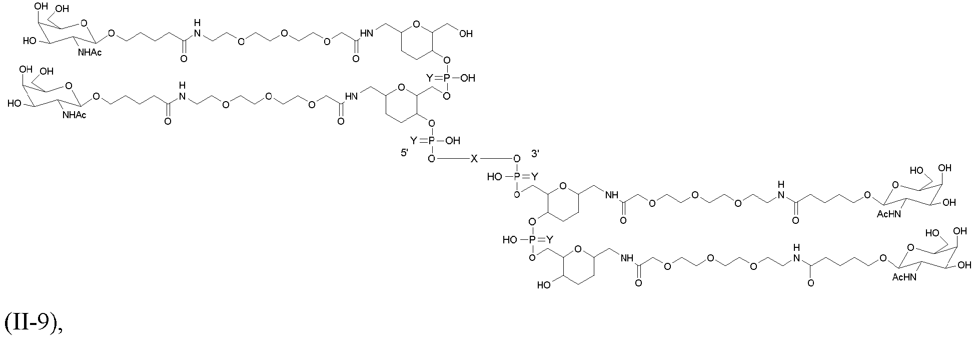 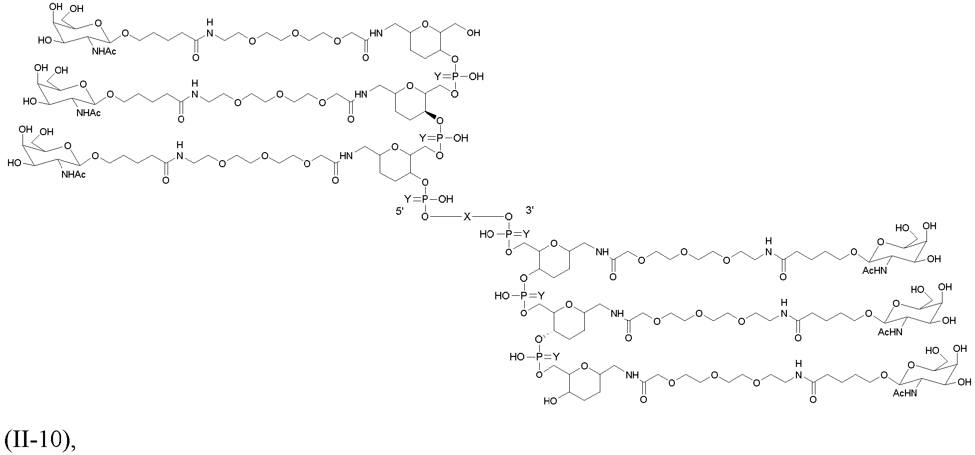 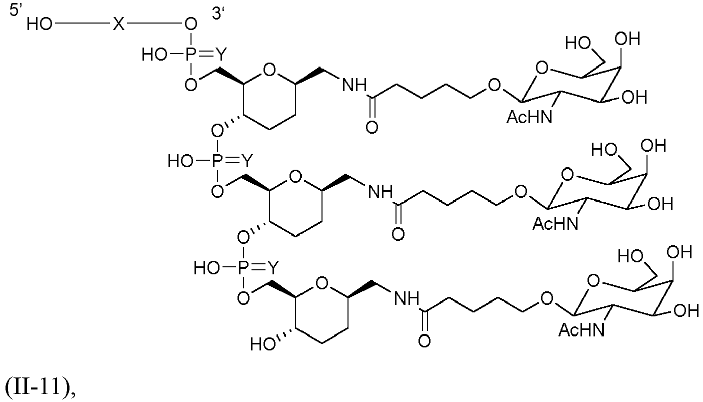 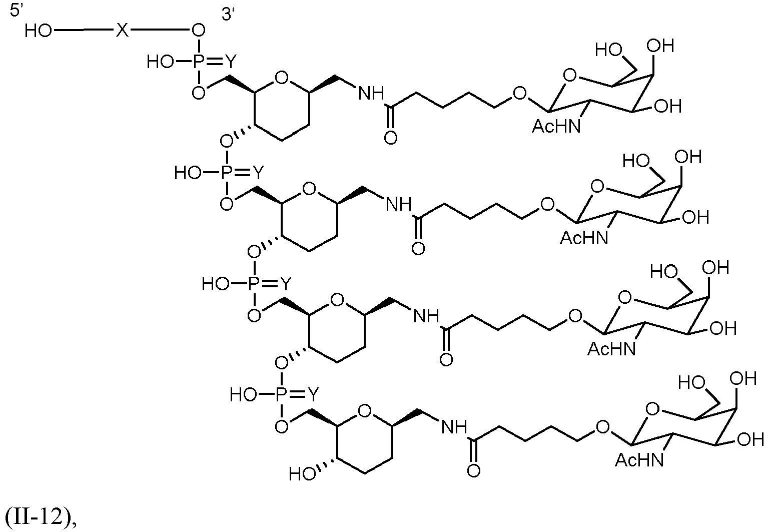 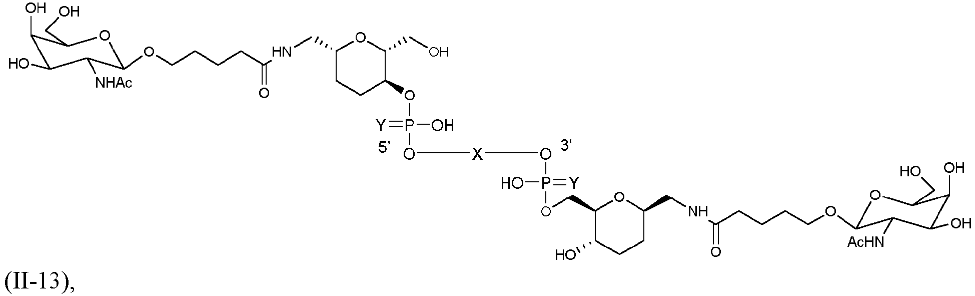 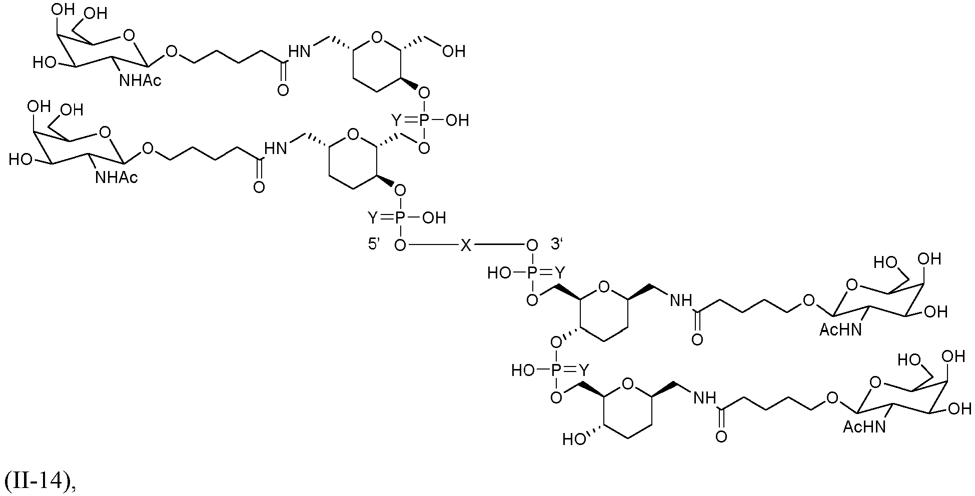 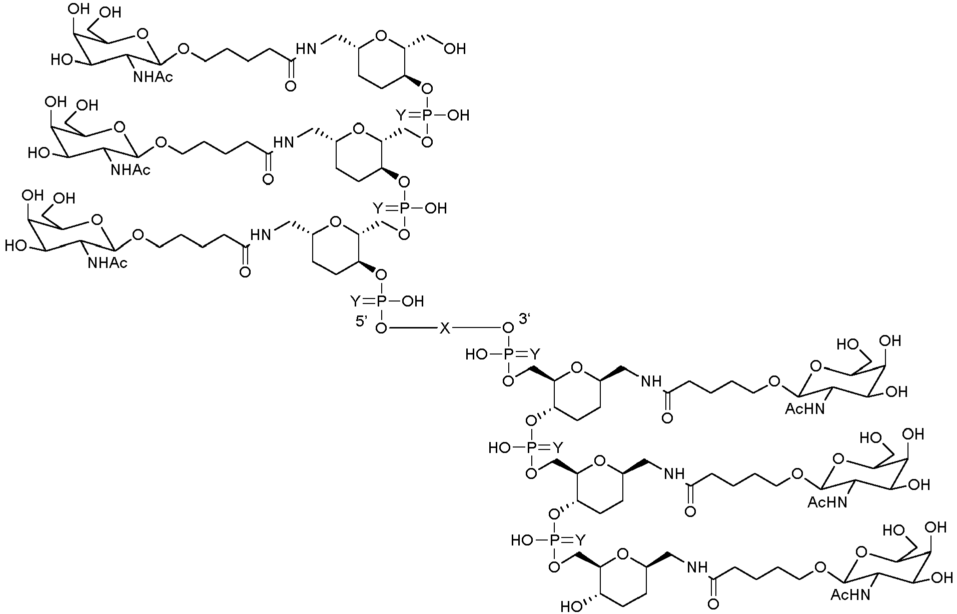 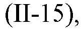 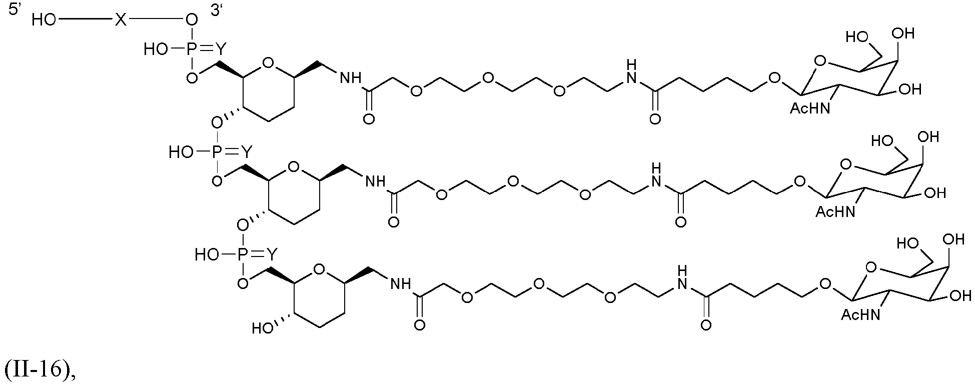 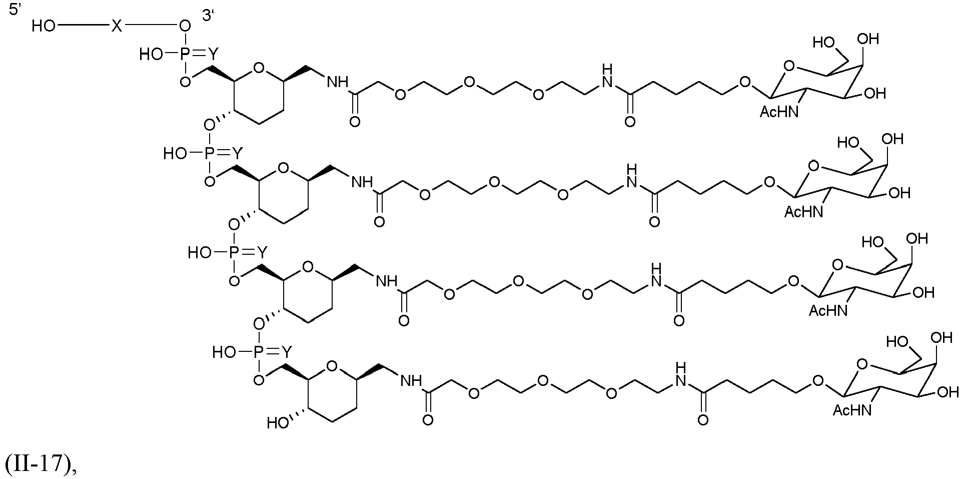 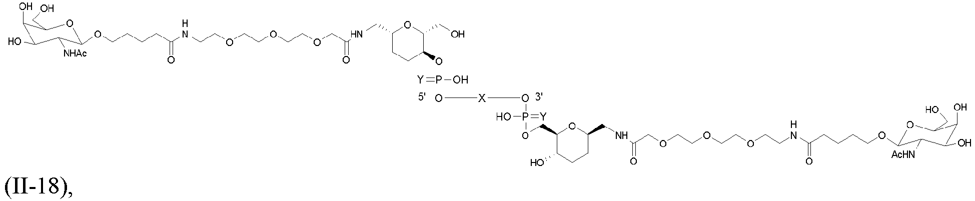 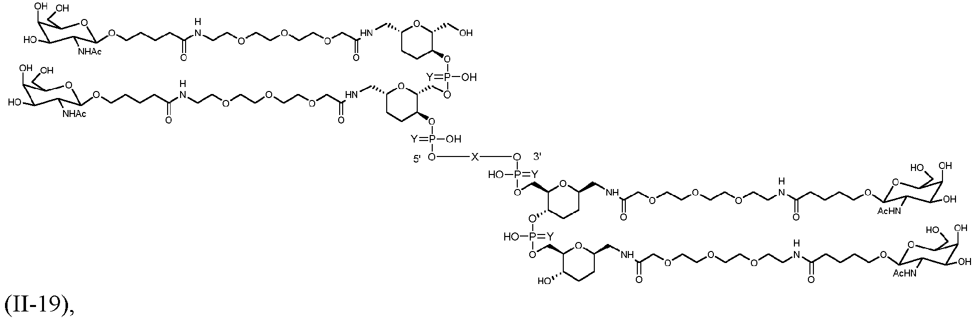 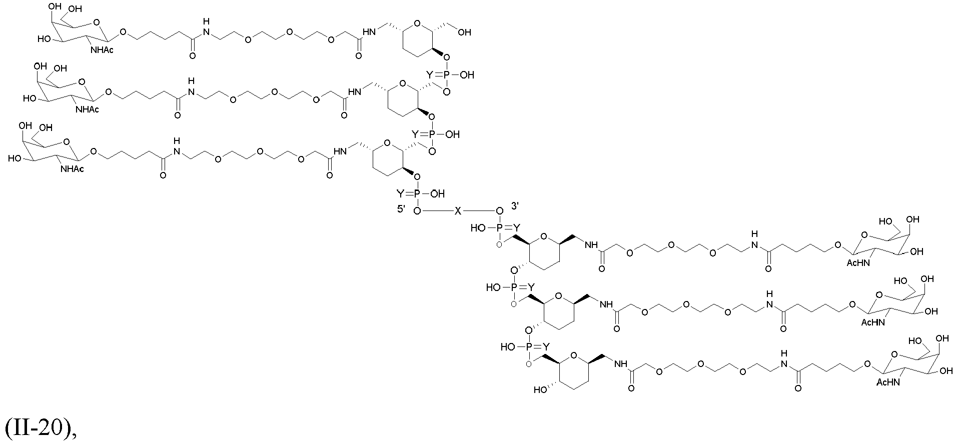 where Y is either O or S.

12. An RNA interference agent, comprising a sense strand and/or an antisense strand, and an oligonucleotide ligand compound or a pharmaceutically acceptable salt thereof according to any one of claims 3-11.

13. The RNA interference agent according to claim 12, comprising antisense oligonucleotides, siRNA, or miRNA.

14. The RNA interference agent according to claim 12, comprising one or more modified nucleotides.

15. The RNA interference agent according to claim 12, wherein one or more nucleotides on the sense and/or antisense strands are modified to form modified nucleotides.

16. The RNA interference agent according to claim 12 is connected to an oligonucleotide ligand compound or a pharmaceutically acceptable salt thereof via a phosphate ester group, thiophosphate ester group, or phosphonate ester group.

17. The RNA interference agent according to claim 12, wherein the oligonucleotide ligand compound or pharmaceutically acceptable salt thereof is coupled to a sense and/or antisense strand.

18. The RNA interference agent according to claim 12, wherein the oligonucleotide ligand compound or pharmaceutically acceptable salt thereof is conjugated to the 5' and/or 3' end of the antisense strand, or to the 5' and/or 3' end of the sense strand.

19. A pharmaceutical composition, comprising a cleavable compound or a pharmaceutically acceptable salt thereof according to claim 1 or 2, or an oligonucleotide ligand compound or a pharmaceutically acceptable salt thereof according to any one of claims 3-11.

20. The use of a cleavable compound or pharmaceutically acceptable salt thereof according to claim 1 or 2, or an oligonucleotide ligand compound or pharmaceutically acceptable salt thereof according to any one of claims 3-11 in the preparation of drugs for the prevention and/or treatment of physiological conditions or diseases caused by the expression of specific genes in liver cells, or physiological conditions or diseases caused by liver cell dysfunction.

21. The use according to claim 20, wherein the use comprises maintaining the contact between liver cells and the cleavable compound or pharmaceutically acceptable salt thereof according to claim 1 or 2, or the oligonucleotide ligand compound or pharmaceutically acceptable salt thereof according to any one of claims 3-11.

22. The use according to claim 20, wherein the cleavable compound breaks at R₂ after delivering the nucleic acid molecule to the target cell.

23. The use of a cleavable compound or a pharmaceutically acceptable salt thereof according to claim 1 or 2, or an oligonucleotide ligand compound or pharmaceutically acceptable salt thereof according to any one of claims 3-11 for targeted binding to nucleic acids and delivery of nucleic acids to human or mammalian liver cells.
